# EUROPEAN PATENT APPLICATION

(11) **EP 4 717 272 A1**
(43) Date of publication of application: **01.04.2026**
(21) Application number: 24797383.7
(22) Date of filing: 23.04.2024
(51) Int. Cl.: A61K 38/44, A61P 9/00, A61P 35/00, A61P 25/28, A23L 33/18

(54) **COMPOSITION COMPRISING PRDX3 AS ACTIVE INGREDIENT FOR PREVENTION OR TREATMENT OF MITOCHONDRIA-RELATED DISEASES AND USE THEREOF**

(30) Priority: 27.04.2023 KR 20230055161
(71) Applicant: Ewha University-Industry Collaboration Foundation, Seoul 03760 (KR)
(72) Inventor: OH, Goo Taeg, Seoul 06520 (KR); SONN, Seong Keun, Seoul 01742 (KR)
(74) Representative: Ostertag & Partner Patentanwälte mbB
(86) International application number: PCT/KR2024/005477
(87) International publication number: WO 2024/225727

(57) **Abstract**

The present invention relates to a pharmaceutical composition comprising a Prdx3 peptide or an analogue thereof as an active ingredient for preventing or treating a mitochondria-related disease and a use thereof. The pharmaceutical composition comprising the Prdx3 peptide or the analogue thereof can be used for preventing or treating a mitochondria-related disease by removing mitophagy and removing alpha-synuclein aggregates.

## Description

### CROSS-REFERENCE TO RELATED APPLICATION

This application claims priority to and the benefit of Korean Patent Application No. 10-2023-0055161, filed on APR 27th, 2023, the disclosure of which is incorporated herein by reference in its entirety.

### [Technical Field]

The present invention relates to a composition for preventing or treating a mitochondria-related disease, comprising Prdx3 as an active ingredient and a use thereof.

### [Background Art]

The heart is a tissue with a high number and density of mitochondria, and adult cardiac mitochondria account for approximately 30% of the total cardiomyocyte volume, mainly supplying energy to compensate for the high adenosine triphosphate (ATP) consumption of the beating heart.

Cardiac mitochondria continuously undergo mitophagy to maintain mitochondrial quality control (MQC), despite mitochondrial damage induced by reactive oxygen species (ROS). Accordingly, mitochondria are the major site of energy and ROS production in the heart, and dysfunction of MQC may cause diastolic dysfunction and is associated with heart failure.

MQC is an essential process for cellular physiology and homeostasis, and is strictly regulated to maintain a healthy mitochondrial network by preventing mitochondrial damage and removing damaged mitochondria.

Mitochondria-specific peroxidase peroxiredoxin 3 (Prdx3) is involved in preventing mitochondrial damage by removing mitochondrial reactive oxygen species, thereby protecting against mitochondrial dysfunction. However, it remains to be studied whether Prdx3 affects the removal of damaged mitochondria, such as by removing mitophagy.

### [Disclosure]

### [Technical Problem]

One aspect of the present invention is to provide a pharmaceutical composition for preventing or treating a mitochondria-related disease, comprising a Prdx3 peptide or an analogue thereof as an active ingredient.

Another aspect of the present invention is to provide a health functional food composition for preventing or improving a mitochondria-related disease, composition comprising a Prdx3 peptide or an analogue thereof as an active ingredient.

Still another aspect of the present invention is to provide a pharmaceutical preparation for preventing or improving a mitochondria-related disease, composition comprising a Prdx3 peptide or an analogue thereof as an active ingredient.

Yet another aspect of the present invention is to provide a method for preventing or treating a mitochondria-related disease, comprising administering a pharmaceutical composition comprising a Prdx3 peptide or an analogue thereof to a subject.

### [Technical Solution]

The inventors of the present invention have confirmed that a Prdx3 peptide or an analogue thereof can be used as an active ingredient for preventing or treating a mitochondria-related disease, and thus completed the present invention.

The present invention provides a pharmaceutical composition for preventing or treating a mitochondria-related disease, comprising a Prdx3 peptide or an analogue thereof as an active ingredient.

The term "Prdx3 (Peroxiredoxin 3)" as used herein refers to a member of the peroxiredoxin family that plays an important role in protecting cells against oxidative stress by detoxifying peroxides. The Prdx3 may be present in mitochondria as an oxidase and may be predominantly present in tissues such as the heart, adrenal gland, liver, and brain.

The inventors of the present invention confirmed that the Prdx3 can maintain mitochondrial activity or remove damage even without a peroxide detoxification effect. In an embodiment, the Prdx3 may act to remove mitophagy, and accordingly, may exhibit a preventive or therapeutic effect on a mitochondria-related disease.

In an embodiment, the Prdx3 may regulate mitochondrial quality through dual antioxidant and chaperone functions.

The term "mitochondrial quality control" as used herein may include protecting mitochondria from reactive oxygen species and removing damaged mitochondria through a mitophagy process.

The term "peptide" as used herein refers to a polymer of amino acids and may include not only natural amino acids but also non-proteinogenic amino acids as components.

The term "peptide analogue" as used herein may include an analogue in which one or more other functional groups are substituted for a side chain or an alpha-amino acid backbone of an amino acid. Examples of side chain- or backbone-modified peptide analogues include hydroxyproline in which a pyrrolidine ring is substituted with a hydroxyl group, and N-methylglycine "peptoid," but are not limited thereto. Types of peptide analogues are known in the art.

In an embodiment, the Prdx3 analogue is characterized in that it is a dominant-negative (DN) mutant of Prdx3. The dominant-negative mutant of Prdx3 may be a form in which cysteine at positions 108 and 229 of Prdx3 are substituted with serine, and such a mutation shows a characteristic of being capable of regulating mitophagy without a ROS removal function of Prdx3.

In an embodiment, the Prdx3 peptide may comprise a chain of 194 amino acids at positions 64 to 257 of the Prdx3 gene and a transit peptide of 63 amino acids at positions 1 to 63 of the Prdx3 gene.

In an embodiment, the Prdx3 peptide may remove mitophagy. In one example, the Prdx3 peptide may regulate sub-mitochondrial localization of PINK1 and prevent mitochondrial damage.

The term "autophagy" as used herein refers to an activity in which a cell degrades its own proteins or eliminates unnecessary cellular components to obtain energy when the cell is in a nutrient-deficient condition. For example, it may be mitophagy.

The term "mitophagy" as used herein refers to selective degradation of mitochondria by autophagy, which is a mitochondria-specific response occurring due to any damage or stress. This is characterized in that it promotes mitochondrial turnover and prevents accumulation of dysfunctional mitochondria that cause cellular degradation, thereby maintaining mitochondria in a healthy state.

That is, when mitophagy does not proceed smoothly, a mitochondria-related disease may occur due to abnormalities within cells. Accordingly, regulating the mitophagy is useful for preventing or treating the disease.

The composition of the present invention, by comprising a Prdx3 peptide or an analogue thereof as an active ingredient, has an effect of improving or treating a disease caused by an autophagy disorder in mitochondria, can contribute to research related thereto, and has an advantage of being applicable as various pharmaceuticals or health functional foods.

In an embodiment, deficiency of the Prdx3 peptide may cause cardiac dysfunction, for example, cardiac dysfunction caused by myocardial infarction (MI).

In an embodiment, the Prdx3 peptide may interact with a PINK1 peptide and regulate mitophagy through degradation of the PINK1 peptide.

The term "PINK1 (PTEN-induced kinase 1)" as used herein refers to a protein localized in the mitochondrial matrix, the N-terminus of which can be cleaved by PARL and MPP through sequential proteolysis. In response to mitochondrial damage, PINK1 accumulates on the outer mitochondrial membrane (OMM) of damaged mitochondria, which can mediate mitophagy.

In an embodiment, deficiency of the Prdx3 peptide may result in reduced mitophagy despite upregulation of PINK1.

In an embodiment, the Prdx3 may inhibit expression or action of Omal, and maintain stability by inhibiting degradation of PINK1 by Omal.

In an embodiment, the Prdx3 may affect localization of Parkin and regulate PINK1-Parkin-mediated mitophagy.

In an embodiment, the Prdx3 may remove an alpha-synuclein aggregate.

The term "alpha-synuclein" as used herein refers to a protein abundantly present in the human brain, and when alpha-synuclein aggregates to form insoluble protofibrils, i.e., aggregates, it acts as a neurotoxin that reduces dopamine and causes Parkinson's disease.

The term "mitochondria-related disease" as used herein may refer to one or more diseases selected from the group consisting of cardiovascular disease, lymphoma, glomerulonephritis, osteoporosis, motor neuron disease, muscular atrophy, Down syndrome, carcinoma, Pick disease, Parkinson syndrome, and Alzheimer's disease.

In an embodiment, the Prdx3 may act on mitochondrial quality control by regulating both protecting mitochondria from reactive oxygen species and removing damaged mitochondria through a mitophagy process. In another embodiment, it was confirmed that a Prdx3-deficient mouse has damaged mitochondria in most tissues of the body, and thus may be an important model mouse for studying mitochondrial function in vivo.

The term "prevention" as used herein refers to all acts of suppressing or delaying the onset of a mitochondria-related disease by administration of the pharmaceutical composition according to the present invention.

The term "treatment" as used herein refers to all acts of improving or beneficially changing symptoms of a mitochondria-related disease by administration of the pharmaceutical composition according to the present invention.

The composition according to the present invention may be used alone for preventing or treating a mitochondria-related disease, or in combination with surgery, radiotherapy, chemotherapy, and biological response modifiers, and preferably may be used in combination with a drug that promotes prevention or treatment of a mitochondria-related disease.

The composition according to the present invention may further comprise a pharmaceutically acceptable carrier. The pharmaceutically acceptable carrier is conventionally used in formulation and may include, but is not limited to, saline, sterile water, Ringer's solution, buffered saline, cyclodextrin, dextrose solution, maltodextrin solution, glycerol, ethanol, and liposomes. In addition, other conventional additives such as antioxidants and buffers may be further included as necessary. Furthermore, diluents, dispersants, surfactants, binders, and lubricants may be additionally added to prepare injectable formulations such as aqueous solutions, suspensions, or emulsions, infusion formulations such as infusion bags, aerosol formulations such as sprays, or pill, capsule, granule, or tablet formulations. Suitable pharmaceutically acceptable carriers and formulation methods may be employed depending on each component using methods disclosed in Remington's reference. The pharmaceutical preparation of the present invention is not particularly limited in dosage form and may be formulated as an injectable, infusion, spray formulation, liquid formulation, or topical formulation.

The composition of the present invention may be administered orally or parenterally (for example, intravenously, subcutaneously, intraperitoneally, or topically including ocular administration) depending on the intended method. The dosage may vary depending on the condition and weight of the patient, the degree of the disease, the type of drug, the route and timing of administration, but may be appropriately selected by a person skilled in the art.

The composition of the present invention may be administered in a pharmaceutically effective amount. The term "pharmaceutically effective amount" as used herein refers to an amount sufficient to treat or diagnose a disease with a reasonable benefit/risk ratio applicable to medical treatment or diagnosis, and the effective dose level may be determined depending on factors such as the type and severity of the disease of the patient, the activity of the drug, the sensitivity to the drug, the time and route of administration, the excretion rate, the treatment period, the concomitant drug, and other factors well known in the medical field. The composition according to the present invention may be administered as a single therapeutic agent or in combination with another therapeutic agent, and may be administered sequentially or simultaneously with a conventional therapeutic agent, either singly or multiply. It is important to administer an amount that provides the maximum effect with the minimum amount without side effects, and this can be readily determined by a person skilled in the art.

Specifically, the effective amount of the composition of the present invention may vary depending on the age, sex, condition, body weight, absorption of the active ingredient in the body, inactivation rate and excretion rate, type of disease, and concomitant drugs of the patient, but generally may be administered daily or every other day in an amount of about 0.001 to 150 mg per kg of body weight, preferably 0.01 to 100 mg, or may be administered in 1 to 3 divided doses per day. However, the dosage may be increased or decreased depending on the route of administration, severity of obesity, sex, body weight, age, etc., and thus the dosage is not intended to limit the scope of the present invention in any way.

The combined administration described herein may be performed in parallel or alternately, and the form of the combined administration may include simultaneous administration of a peptide or peptide analogue with another compound or separate administration thereof.

In addition, the present invention provides a health functional food composition for preventing or improving a mitochondria-related disease, composition comprising a Prdx3 peptide or an analogue thereof as an active ingredient. The term "improvement" as used herein may refer to all acts of at least reducing parameters related to a condition to be treated, for example, the degree of symptoms. The health functional food may be used before or after the onset of the disease, or simultaneously with or separately from a therapeutic agent for treatment, in order to prevent or improve cerebrovascular diseases.

In the health functional food, the active ingredient may be directly added to food or used together with other foods or food components, and may be appropriately used according to conventional methods. The mixing amount of the active ingredient may be suitably determined depending on its intended purpose (prevention or improvement). Generally, when manufacturing food or beverages, the health functional food may be added in an amount of about 15% by weight or less, more specifically about 10% by weight or less, based on the raw material. However, in the case of long-term intake for health and hygiene purposes or for health control, the amount may be within the above range.

The health functional food may further comprise one or more of a carrier, diluent, excipient, and additive, and may be formulated into one selected from the group consisting of tablets, pills, powders, granules, capsules, and liquid formulations. Foods to which a compound according to one aspect may be added include various foods, powders, granules, tablets, capsules, syrups, beverages, gums, teas, vitamin complexes, and health functional foods.

Specific examples of the carrier, excipient, diluent, and additive may include at least one selected from the group consisting of lactose, dextrose, sucrose, sorbitol, mannitol, erythritol, starch, acacia gum, calcium phosphate, alginate, gelatin, calcium phosphate, calcium silicate, microcrystalline cellulose, polyvinylpyrrolidone, cellulose, methylcellulose, water, sugar syrup, methylcellulose, methyl hydroxybenzoate, propyl hydroxybenzoate, talc, magnesium stearate, and mineral oil.

The health functional food may contain other components as essential ingredients without particular limitation in addition to the active ingredient. For example, like a conventional beverage, it may further contain various flavoring agents or natural carbohydrates as additional components. Examples of the natural carbohydrates include monosaccharides such as glucose and fructose; disaccharides such as maltose and sucrose; polysaccharides such as dextrin and cyclodextrin; and sugar alcohols such as xylitol, sorbitol, and erythritol. In addition to the above, natural flavoring agents (such as thaumatin, stevia extracts (for example, rebaudioside A, glycyrrhizin)) and synthetic flavoring agents (such as saccharin, aspartame) may be advantageously used. The proportion of the natural carbohydrates may be suitably determined by a person skilled in the art.

In addition, the health functional food according to one aspect may contain various nutrients, vitamins, minerals (electrolytes), synthetic flavoring agents, and natural flavoring agents, flavoring agents, coloring agents, and thickening agents (such as cheese, chocolate), pectic acid and salts thereof, alginic acid and salts thereof, organic acids, protective colloidal thickeners, pH regulators, stabilizers, preservatives, glycerin, alcohol, and carbonating agents used in carbonated beverages. These ingredients may be used independently or in combination, and the proportions of these additives may also be suitably selected by a person skilled in the art.

In an embodiment, the Prdx3 peptide or an analogue thereof may act on autophagy in mitochondria and apoptosis to exhibit preventive or improvement effects on a mitochondria-related disease.

The present invention also provides a pharmaceutical preparation for preventing or treating a mitochondria-related disease, comprising a Prdx3 peptide or an analogue thereof as an active ingredient.

In one implementation of the present invention, the pharmaceutical preparation may be an injectable formulation, infusion formulation, spray formulation, or liquid formulation.

The present invention also provides a method for preventing or treating a mitochondria-related disease, comprising administering a pharmaceutical composition comprising a Prdx3 peptide or an analogue thereof to a subject.

The term "subject" as used herein refers to a target requiring treatment of a disease, and more specifically refers to a mammal such as a human or non-human primate, mouse, rat, dog, cat, horse, or cow.

The present invention also provides a use of a Prdx3 peptide or an analogue thereof for preventing or treating a mitochondria-related disease.

The present invention also provides a use of a composition comprising a Prdx3 peptide or an analogue thereof as an active ingredient for preventing or treating a mitochondria-related disease.

The present invention also provides a use of a Prdx3 peptide or an analogue thereof for the manufacture of a medicament for preventing or treating a mitochondria-related disease.

The present invention also provides a use of a Prdx3 peptide or an analogue thereof and macrophages as active ingredients for the manufacture of a medicament for preventing or treating a mitochondria-related disease.

The above-described features may be used in combination, and the fact that the features are described in different dependent claims does not mean that they cannot be used in combination.

The present invention also provides a Prdx3 knockout mouse model in which exon 1 to exon 4 in a 5'-UTR region of a Prdx3 gene are deleted.

In an embodiment, the mouse model may be a Parkinson's disease mouse model.

The present invention also provides a method for preparing a Prdx3 knockout mouse model, comprising deleting exon 1 to exon 4 in a 5'-UTR region of a Prdx3 gene. In one specific embodiment, exons 1 to 6 of the Prdx3 gene may comprise sequences represented by SEQ ID NOs: 7 to 12.

In an embodiment, the deletion of exon 1 to exon 4 of the 5'-UTR region in the Prdx3 gene may be performed by homologous recombination.

In an embodiment, the deletion may be carried out by replacing a nucleotide sequence comprising exon 1 to exon 4 of the 5'-UTR region in the Prdx3 gene with a construct comprising a neomycin gene.

In the present invention, the construct may be one in which a part of an intron of the 5'-UTR of exon 1 of the Prdx3 gene is inserted into the 5'-UTR of an exogenous gene, neomycin, and a part of an intron of the 3'-UTR of exon 4 of the Prdx3 gene is recombined into the 3'-UTR.

In an embodiment, the replacement with the construct may be carried out by electroporation.

### [Advantageous Effects]

The pharmaceutical composition comprising a Prdx3 peptide or an analogue thereof of the present invention can be used for preventing or treating a mitochondria-related disease (such as Parkinson's disease) by removing mitophagy and removing alpha-synuclein aggregates through the Prdx3 peptide or the analogue thereof. However, the advantageous effects of the present invention are not limited to the above-described effects, and it should be understood that all effects that can be inferred from the constitution of the invention described in the detailed description or the claims are encompassed within the scope of the present invention.

### [Description of Drawings]

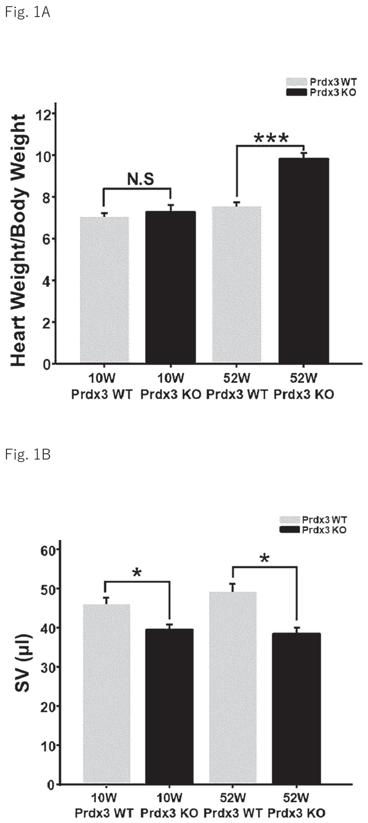

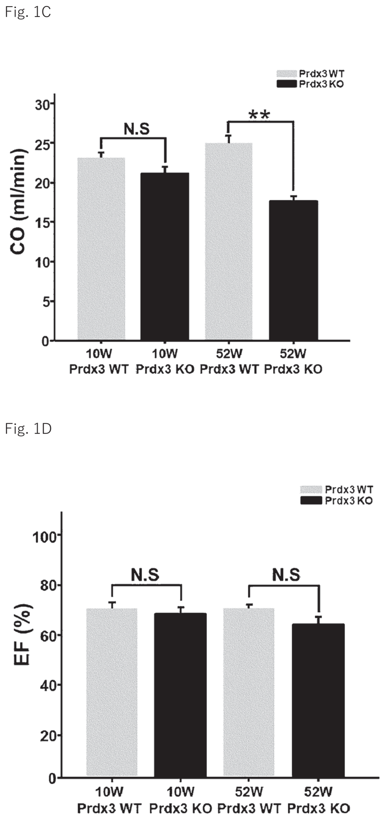

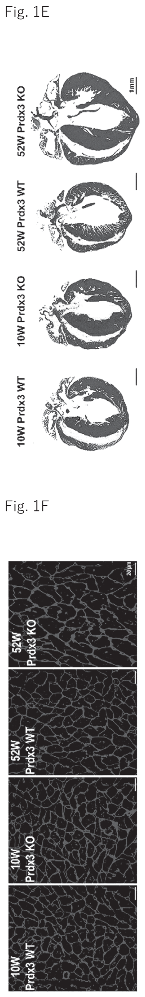

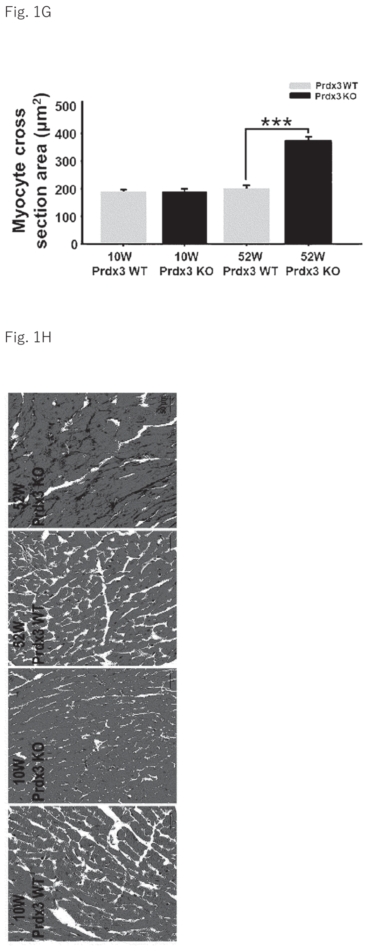

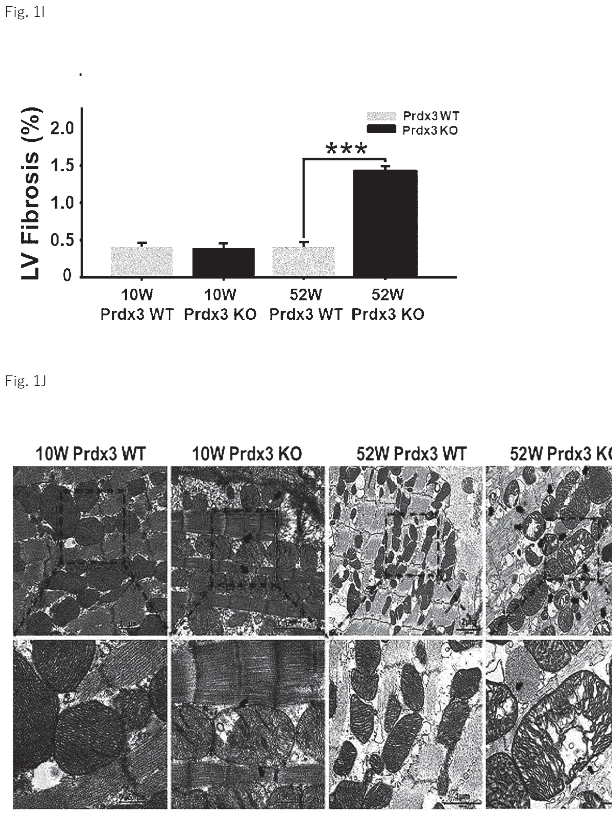

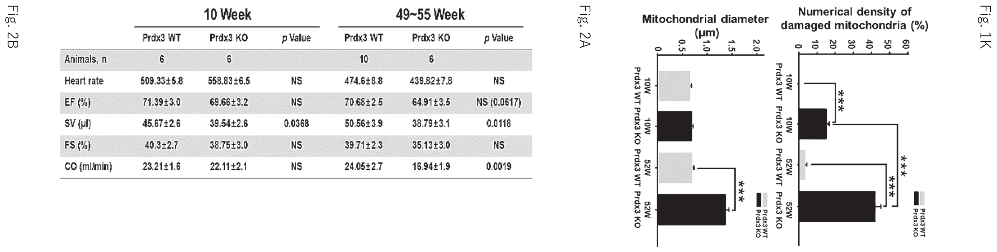

FIG. 1 shows that peroxiredoxin 3 (Prdx3) deficiency causes cardiac hypertrophy with enlarged and damaged mitochondria. FIG. 1A shows the heart weight/body weight ratio between Prdx3 wild-type (Prdx3 WT) and Prdx3 knockout (Prdx3 KO) mice at 10 weeks (n = 5/group) and 52 weeks of age (n = 6/group). FIGS. 1B to 1D show the results of echocardiographic cardiac function analysis in Prdx3 WT mice at 10 weeks (n = 6) and 52 weeks (n = 10), and Prdx3 KO mice at 10 weeks (n = 6) and 52 weeks (n = 6). Stroke volume (SV) (1B), cardiac output (CO) (1C), and ejection fraction (EF) (1D) were measured in the left ventricular (LV) long-axis view using Simpson's monoplane method of disk approach. FIG. 1E shows hematoxylin and eosin staining results of Prdx3 WT and Prdx3 KO hearts (scale bar, 1 mm). FIGS. 1F and 1G show visualization of cell boundaries and quantification of LV fibrosis by wheat germ agglutinin staining of LV muscle sections (scale bar, 20 µm). FIGS. 1H and 1I show Masson's trichrome staining of LV muscle sections and quantification of cardiac fibrosis (scale bar, 50 µm). FIGS. 1J and 1K show representative electron microscopy images of hearts from Prdx3 WT and Prdx3 KO mice and quantification of damaged mitochondria (arrows). For 10-week-old Prdx3 WT and Prdx3 KO mice, the scale bar is 0.5 µm (boxed area) or 1 µm. For 52-week-old Prdx3 WT and Prdx3 KO mice, the scale bar is 1 µm (boxed area) or 2 µm. *P < 0.05, **P < 0.01, and ***P < 0.001.

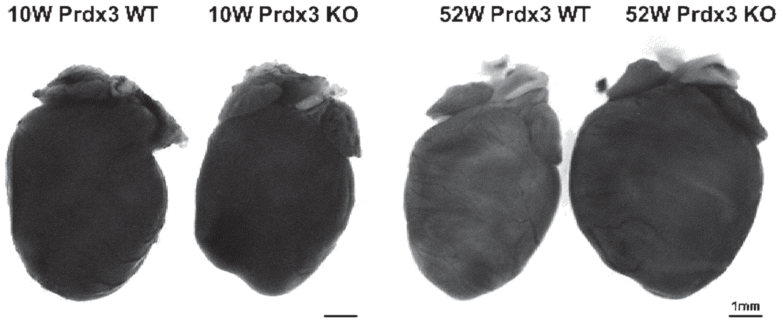

FIG. 2 shows transthoracic echocardiographic of Prdx3 WT and Prdx3 KO mice. FIG. 2A shows pooled echocardiographic data for 10-week-old Prdx3 WT (n = 6) and 52-week-old Prdx3 WT (n = 10), and 10-week-old Prdx3 KO (n = 6) and 52-week-old Prdx3 KO mice (n = 6). EF (ejection fraction), SV (stroke volume), FS (fractional shortening), and CO (cardiac output) are shown. FIG. 2B shows heart images of 10-week-old Prdx3 WT, 52-week-old Prdx3 WT, 10-week-old Prdx3 KO, and 52-week-old Prdx3 KO mice. (scale bar, 1 mm).

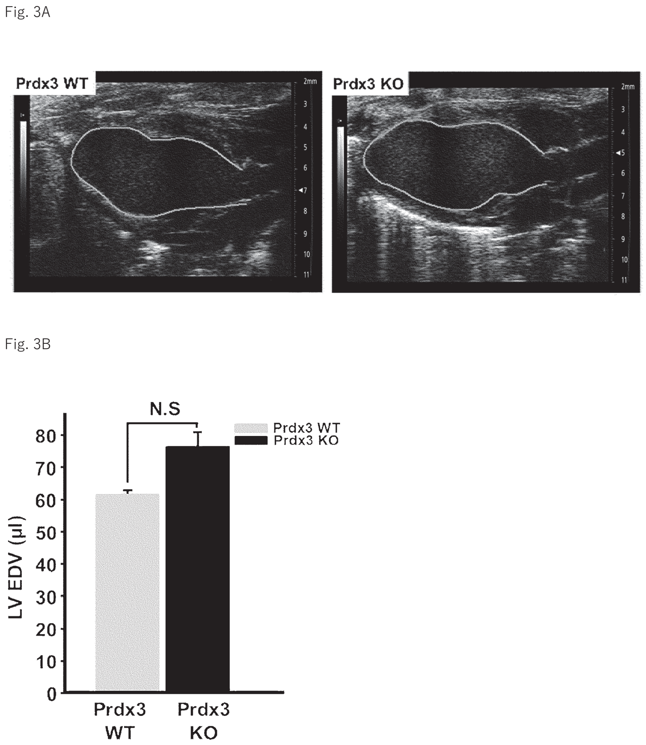

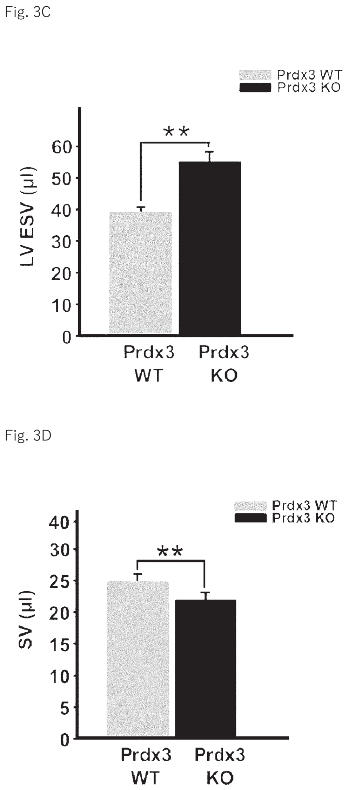

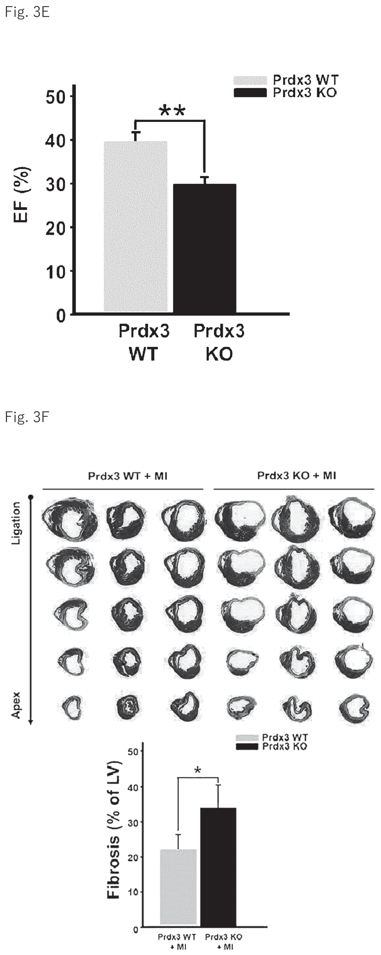

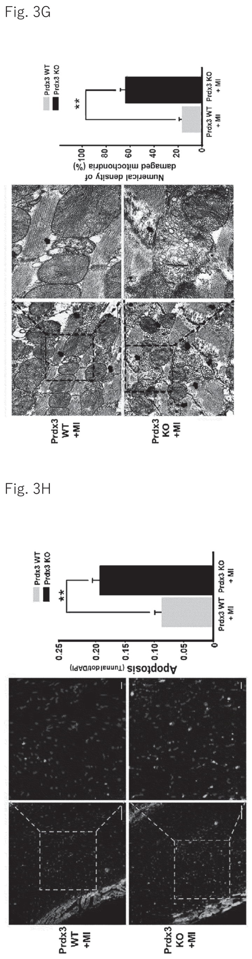

FIG. 3 shows that Prdx3 deficiency accelerates left ventricular (LV) remodeling and cardiac failure after myocardial infarction (MI). FIG. 3A shows echocardiographic cardiac function analysis of Prdx3 WT (n = 7) and Prdx3 KO (n = 8) mice at 15 days after MI. FIG. 3B shows end-diastolic volume (EDV), FIG. 3C shows end-systolic volume (ESV), FIG. 3D shows stroke volume (SV), and FIG. 3E shows ejection fraction (EF), each measured from LV long-axis views using Simpson's monoplane method of disks approach. FIG. 3F shows serial sections of Masson's trichrome-stained hearts from Prdx3 WT and Prdx3 KO mice 15 days after MI (fibrosis percentage was calculated relative to the total LV area). FIG. 3G shows representative electron micrographs of the hearts of WT and Prdx3 KO mice at 1 day after MI. Arrows indicate damaged mitochondria. (scale bar, 0.5 µm (boxed areas) or 2 µm). FIG. 3H shows TUNEL analysis results of the infarcted areas of Prdx3 WT (n = 5) and Prdx3 KO (n = 5) mice at 1 day after MI (scale bar, 10 µm (boxed areas) or 50 µm). *P < 0.05 and **P < 0.01.

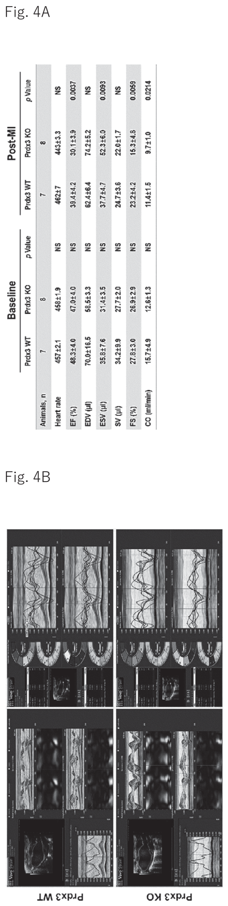

FIG. 4 shows transthoracic echocardiographic of Prdx3 WT and Prdx3 KO mice after MI. FIG. 4A shows pooled echocardiographic data of Prdx3 WT (n = 7) and Prdx3 KO (n = 8) mice. FIG. 4B shows left ventricular (LV) function using VevoStrain software.

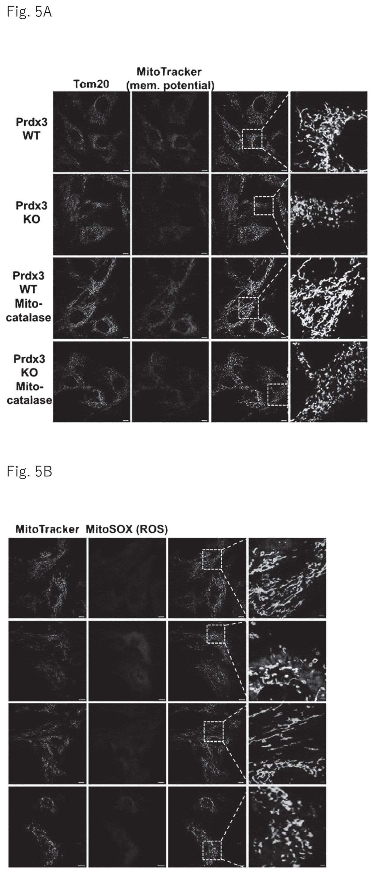

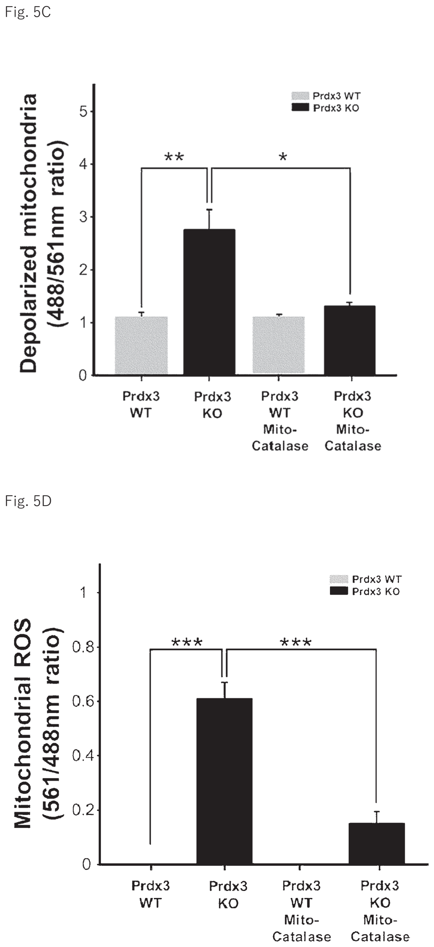

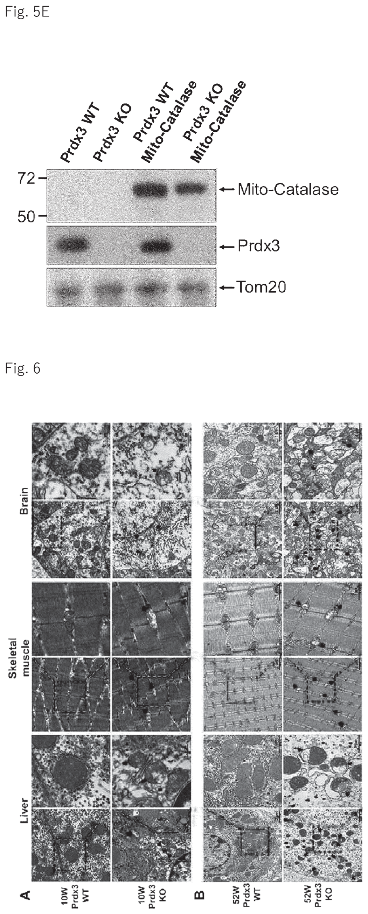

FIG. 5 shows that increasing mitochondrial damage is attributed to reactive oxygen species (ROS) accumulation by Prdx3 deficiency. FIGS. 5A and 5B show representative low- and high-magnification images of boxed areas of Prdx3 WT and Prdx3 KO mouse embryonic fibroblasts (MEFs). MEFs were infected for 24 hours with mito-catalase adenovirus and immunostained with an antibody against Tom20 plus 100 nM MitoTracker (FIG. 5A) or 150 nM MitoTracker plus 5 µM MitoSOX (FIG. 5B) for 25 minutes (scale bar, 2 µm (boxed areas) or 10 µm). FIGS. 5C and 5D show quantification of depolarized (damaged) mitochondria (FIG. 5C) or mitochondrial ROS levels (FIG. 5D) in Prdx3 WT and Prdx3 KO MEFs (n = 25-33 cells). FIG. 5E shows immunoblotting of whole-cell lysates of Prdx3 WT and Prdx3 KO MEFs infected for 24 hours with mito-catalase adenovirus using catalase, Prdx3, and Tom20 antibodies (**P < 0.01). Data (5A to 5E) represent three independent experiments.
FIG. 6 shows that Prdx3 deficiency increases mitochondrial damage. FIGS. 6A and 6B show representative electron micrographs of liver, skeletal muscle (soleus), and brain (dentate gyrus region) tissues from 10-week-old Prdx3 wild-type (Prdx3 WT), 52-week-old Prdx3 WT, 10-week-old Prdx3-deficient (Prdx3 KO), and 52-week-old Prdx3 KO mice. Arrows indicate damaged mitochondria (scale bar, 0.5 µm (boxed areas) or 1 µm for 10-week-old mice, 1 µm (boxed areas) or 2 µm for 52-week-old mice).

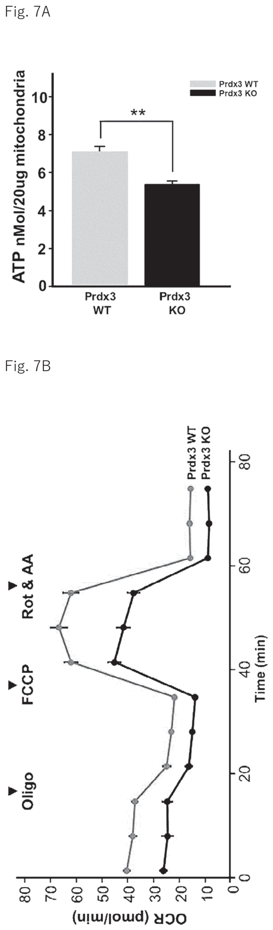

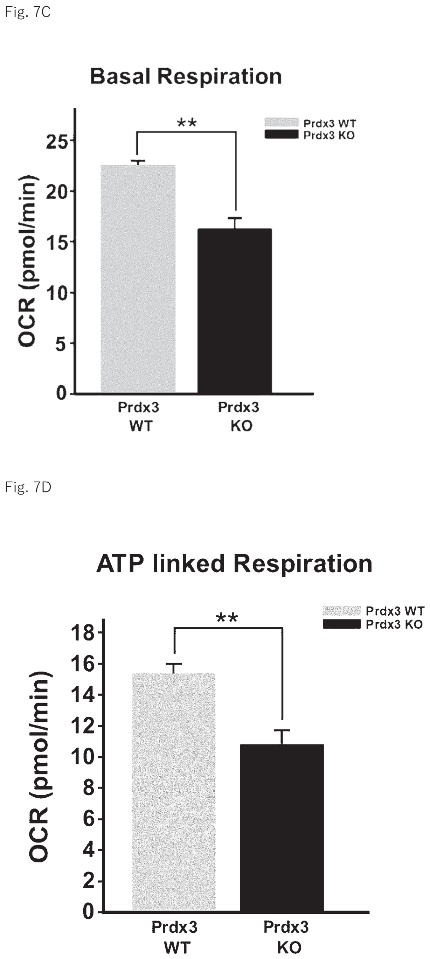

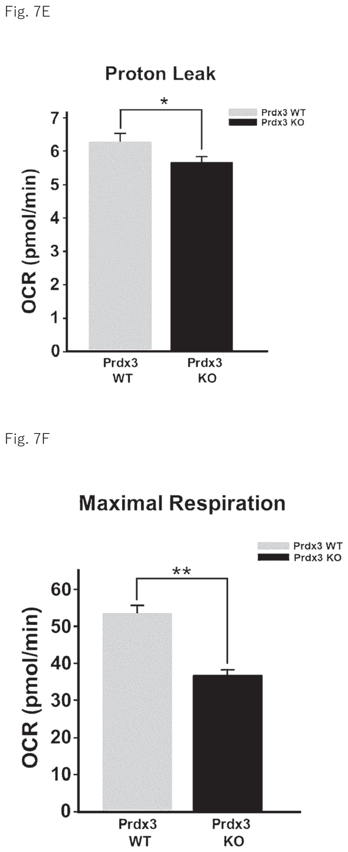

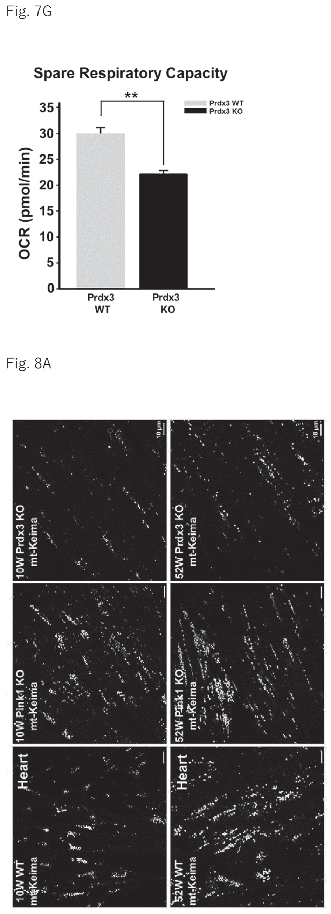

FIG. 7 shows that Prdx3 deficiency causes cardiac mitochondrial dysfunction. FIG. 7A shows ATP production of Prdx3 WT and Prdx3-deficient (Prdx3 KO) cardiomyocytes. FIG. 7B shows representative tracing of oxygen consumption rate (OCR, pMoles/min) of Prdx3 WT and Prdx3-deficient (Prdx3 KO) cardiomyocytes . Arrowheads indicate the time points at which oligomycin (Oligo, 1.5 µM), FCCP (1 µM), and Rot & AA (rotenone and antimycin, 0.5 µM each) were injected into 1 × 10⁴ cells. FIG. 7C shows OCR values of basal respiration per 1 × 10⁴ cells, FIG. 7D OCR values of ATP-linked respiration, FIG. 7E OCR values of proton leak, FIG. 7F OCR values of maximal respiration, and FIG. 7G OCR values of spare respiratory capacity (*P < 0.05, **P < 0.01). Data (7A to 7G) represent three independent experiments.

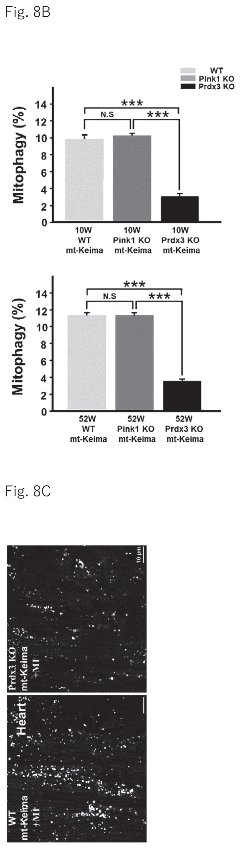

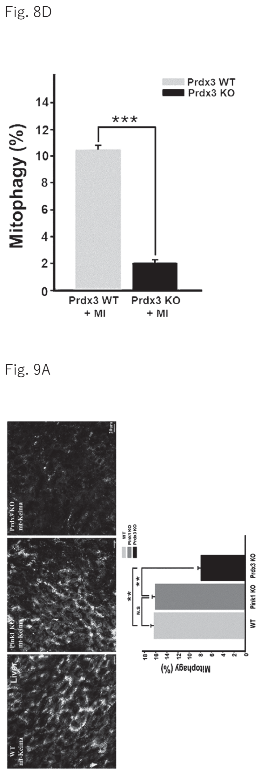

FIG. 8 shows that Prdx3 deficiency reduces mitophagy in the heart. FIGS. 8A and 8B show representative images of mitophagy (FIG. 8A) and quantification graphs (FIG. 8B) in hearts of 10-week-old Prdx3 WT (n = 9), 52-week-old Prdx3 WT (n = 6), 10-week-old Pink1 KO (n = 7), 52-week-old Pink1 KO (n = 6), 10-week-old Prdx3 KO (n = 8), and 52-week-old Prdx3 KO (n = 6) mt-Keima mice (scale bar, 10 µm). FIGS. 8C and 8D show representative images (FIG. 8C) and quantification graphs (FIG. 8D) of mitophagy in infarcted hearts of Prdx3 WT (n = 5) and Prdx3 KO mt-Keima mice (n = 7) 1 day after MI (scale bar, 10 µm, ***P < 0.001).

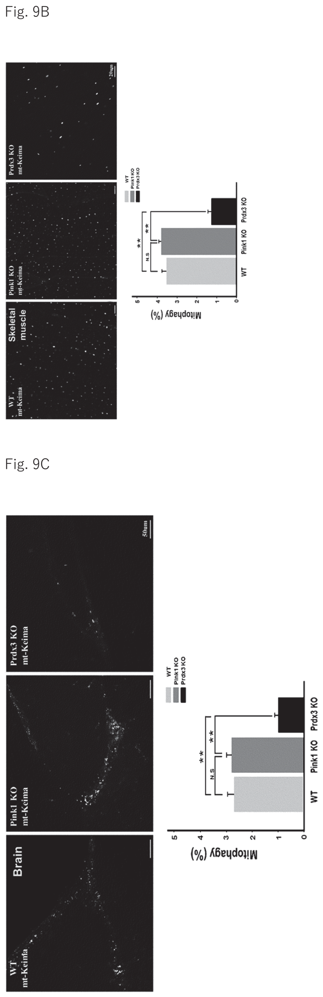

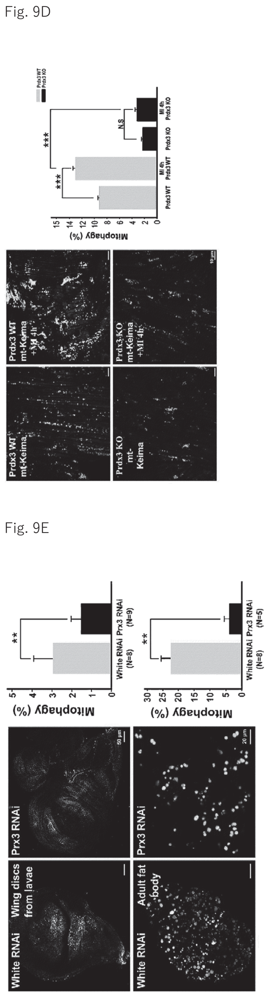

FIG. 9 shows that mitophagy is decreased in the absence of Prdx3 in vivo. FIGS. 9A to 9C show representative images and quantification of mitophagy in the liver (FIG. 9A), skeletal muscle (soleus) (FIG. 9B), and brain tissue (dentate gyrus region) (FIG. 9C) of wild-type (n = 9), Pink1-deficient (KO) mitochondria-targeted Keima (mt)-Keima (n = 7), and Prdx3-deficient (KO) mt-Keima mice (n = 8) (scale bar, 20 µm for liver and skeletal muscle, 50 µm for brain). FIG. 9D shows representative images and quantification of mitophagy in infarcted hearts of Prdx3 WT (n = 6), Prdx3 KO (n = 5), Prdx3 WT mt-Keima (n = 6), and Prdx3 KO mt-Keima mice (n = 6) at 4 hours after MI (scale bar, 10 µm). FIG. 9E shows representative confocal images presented the level of mitophagy in wing disks from mt-Keima Drosophila third instar larvae and adult fat bodies expressing control white RNAi or Prx3 RNAi (da-GAL4>mt-Keima;white RNAi, da-GAL4>mt-Keima;Prx3 RNAi). The levels of mitophagy were quantified using multiple tissue samples (e.g., white RNAi, n = 8; adult fat body white RNAi, n = 8; larvae [Prx3 RNAi], n = 9; adult fat bodies [Prx3 RNAi], n = 5) (scale bar, 20 or 50 µm, **P < 0.01).

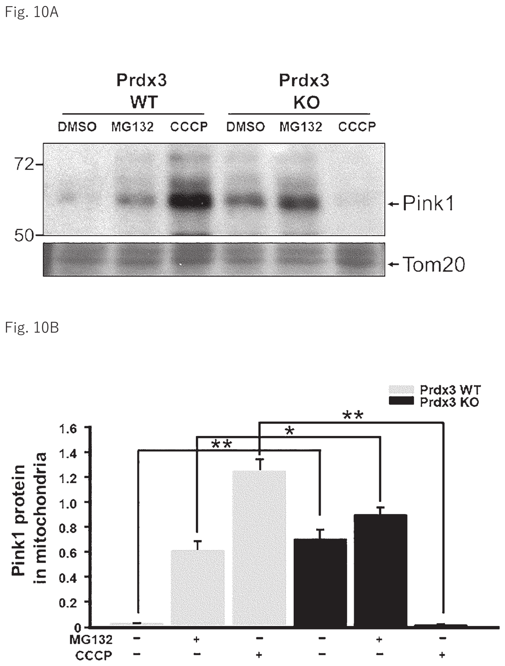

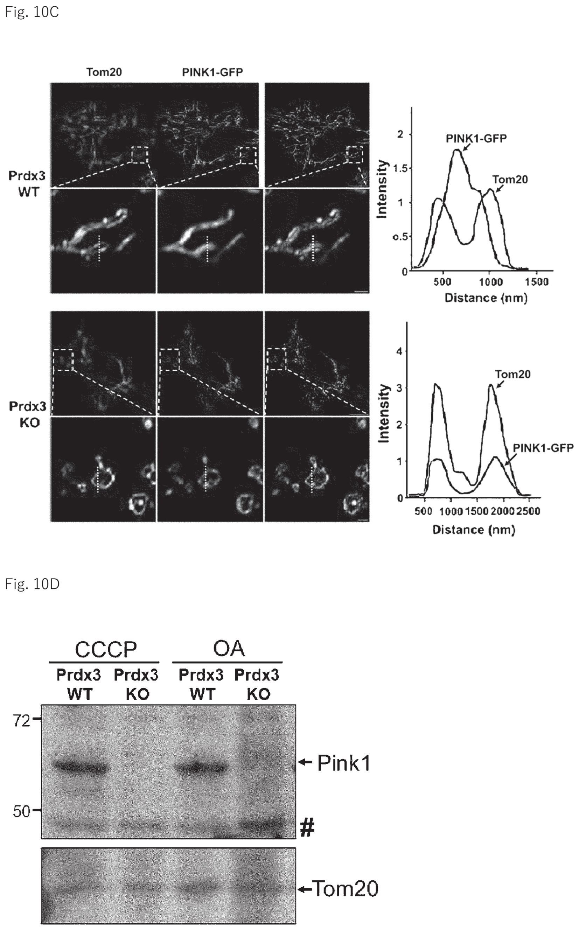

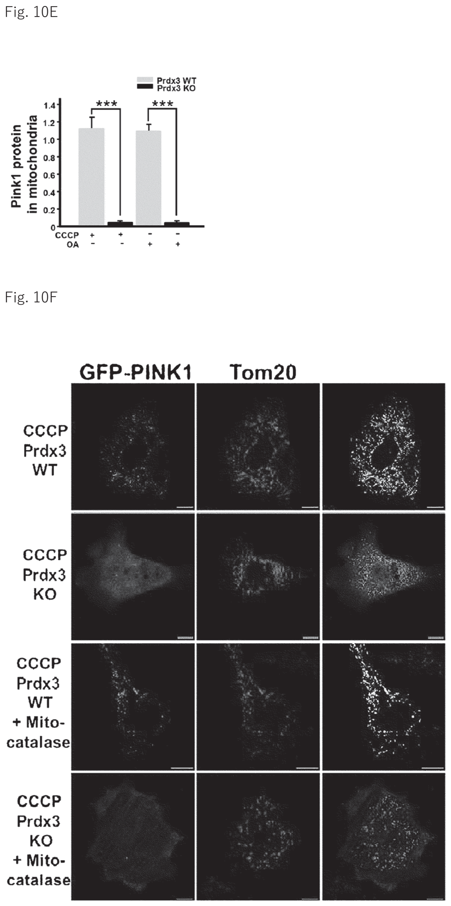

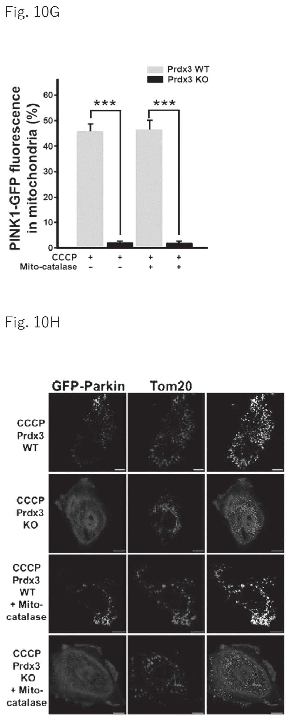

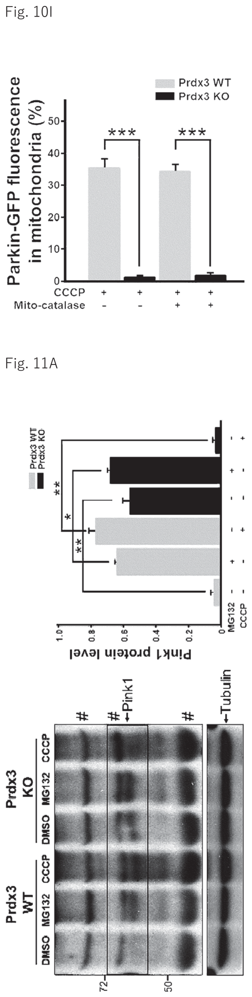

FIG. 10 shows that Prdx3 is required for PINK1-Parkin-mediated mitophagy. FIGS. 10A and 10B show western blot analysis (FIG. 10A) and quantification (FIG. 10B) of PINK1 expression in mitochondrial fractions of Prdx3 WT and Prdx3 KO mouse embryonic fibroblasts (MEFs) treated for 4 hours with DMSO, 20 µM MG132, or 10 µM carbonyl cyanide m-chlorophenylhydrazone (CCCP). The mitochondrial fractions were immunoblotted with PINK1 and Tom20 antibodies. FIG. 10C shows immunostaining results of Prdx3 WT and Prdx3 KO MEFs transfected with PINK1-GFP using Tom20 antibody. Graphs show fluorescence intensities of Tom20 and PINK1-GFP measured along the dotted line (scale bar, 1 µm (boxed areas) or 10 µm). FIGS. 10D and 10E show western blot analysis results (FIG. 10D) and quantification graphs (FIG. 10E) of PINK1 expression in mitochondrial fractions of Prdx3 WT and Prdx3 KO MEFs treated for 4 hours with 10 µM CCCP or 2.5 µM oligomycin and 250 nM antimycin A (OA). The mitochondrial fractions were immunoblotted with PINK1 and Tom20 antibodies. Hashes indicate non-specific bands. FIGS. 10F to 10I show immunostaining results and quantification graphs of PINK1-GFP (FIGS. 10F and 10G) and Parkin-GFP (FIGS. 10H and 10I) levels in Prdx3 WT and Prdx3 KO MEFs. MEFs were infected for 24 hours with mito-catalase adenovirus, transfected with PINK1-GFP or Parkin-GFP, and treated with DMSO or 10 µM CCCP for 4 hours, followed by immunostaining with Tom20 antibody (scale bar, 10 µm, *P < 0.05 and **P < 0.01). Data (10A to 10I) represent three independent experiments.

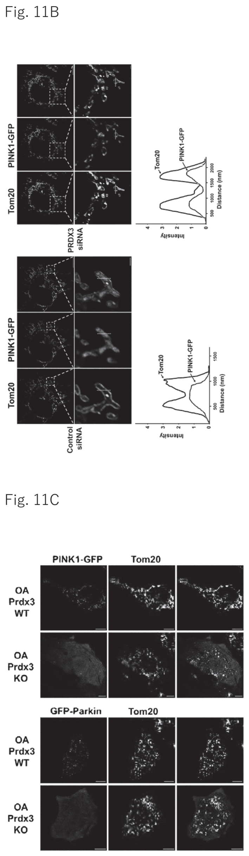

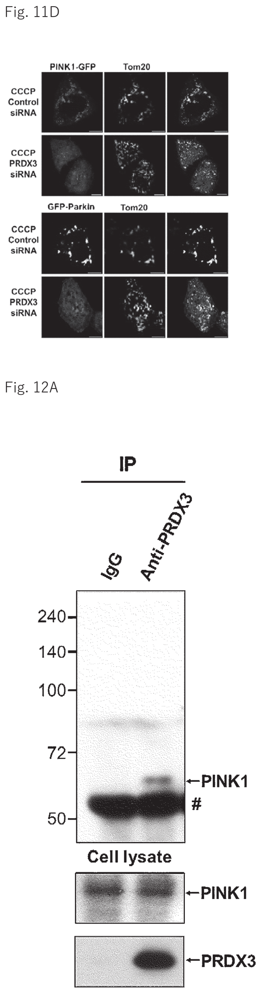

FIG. 11 shows that peroxiredoxin 3 (PRDX3) regulates the recruitment of PINK1 from the outer mitochondrial membrane (OMM) to the matrix and PINK1-Parkin-mediated mitophagy. FIG. 11A shows western blot analysis and quantification of PINK1 levels in cell lysates from Prdx3 wild-type (Prdx3 WT) and Prdx3-deficient (Prdx3 KO) mouse embryonic fibroblasts (MEFs) treated for 4 h with DMSO, 20 µM MG132, or 10 µM carbonyl cyanide m-chlorophenyl hydrazone (CCCP). The cell lysates were immunoblotted with antibodies against PINK1 and tubulin (*P < 0.05 and **P < 0.01). FIG. 11B shows immunostaining of HeLa cells transfected with control or PRDX3 siRNA using a Tom20 antibody. Graphs present fluorescent intensities of Tom and PINK1-GFP measured at the dotted line. FIG. 11C shows immunostaining of Prdx3 WT and Prdx3 KO MEFs transfected with PINK1-GFP or Parkin-GFP and treated with DMSO or 2.5 µM oligomycin/250 nM antimycin A (OA), using a Tom20 antibody (scale bar, 10 µm). FIG. 11D shows immunostaining of PRDX3 siRNA- or control siRNA-treated HeLa cells transfected with PINK1-GFP or Parkin-GFP and treated with DMSO or 10 µM CCCP for 4 h with Tom20 antibody (scale bar, 10 µm; boxed areas scale bar, 1 µm).

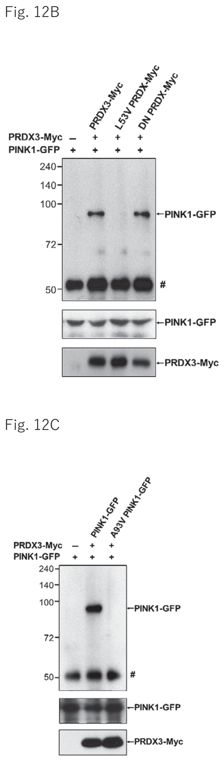

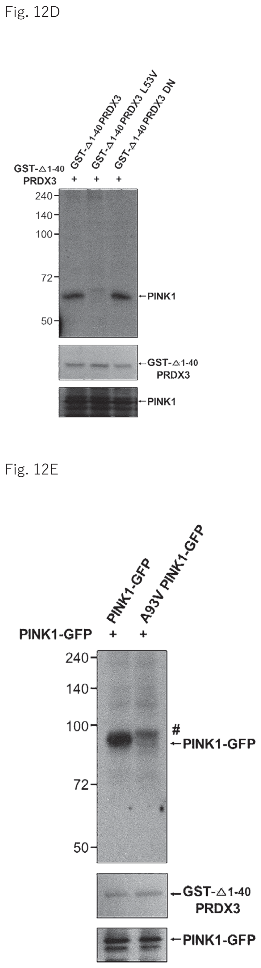

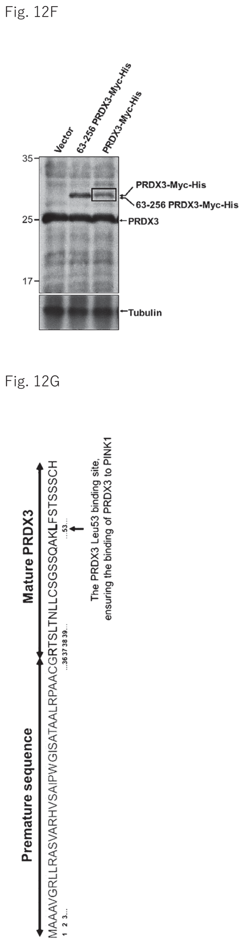

FIG. 12 shows that peroxiredoxin 3 (PRDX3) interacts with PINK1. FIG. 12A shows interaction between endogenous PINK1 and PRDX3. Whole-cell lysates (WCLs) of 293T cells were immunoprecipitated with control IgG and anti-Prdx3 antibody then blotted with anti-PINK1 antibody. FIG. 12B shows subsequent immunoblotting of WCLs from 293T cells co-transfected with various combinations of constructs expressing PINK1-GFP, PRDX3-Myc, L53V PRDX3-Myc, and dominant-negative (DN; Cys108Ser, Cys229Ser) PRDX3-Myc, using anti-Myc beads and a GFP-specific antibody. Hashes indicate IgG. FIG. 12C shows subsequent immunoblotting of WCLs from 293T cells co-transfected with combinations of constructs expressing PINK1-GFP, A93V PINK1-GFP, and PRDX3-Myc, using anti-Myc beads and a GFP-specific antibody. Hashes indicate IgG. FIG. 12D shows immunoprecipitation using GST-Δ1-40PRDX3, GST-Δ1-40PRDX3 L53V, or GST-Δ1-40PRDX3 DN conjugated beads with WCLs from 293T cells transfected with a construct expressing PINK1-GFP, followed by immunoblotting with PINK1- or GFP-specific antibodies. Hashed bands indicate non-specific bands. FIG. 12E shows immunoprecipitation using GST-Δ1-40PRDX3 with WCLs from 293T cells transfected with constructs expressing PINK1-GFP or A93V PINK1-GFP, followed by immunoblotting with PINK1- or GFP-specific antibodies. Hashes denote non-specific bands. FIG. 12F shows immunoblotting of WCLs from 293T cells transfected with Myc-His, 63-256 PRDX3-Myc-His, or 1-256 PRDX3-Myc-His expression vectors using PRDX3 and tubulin antibodies. FIG. 12G shows mass spectrometric analysis of 1-256 PRDX3-Myc-His (box in panel f), the presequence (residues 1-36 of the PRDX3 mitochondrial targeting sequence), and the predicted MPP cleavage site of mature PRDX3. Data (12A-12E) represent three independent experiments.

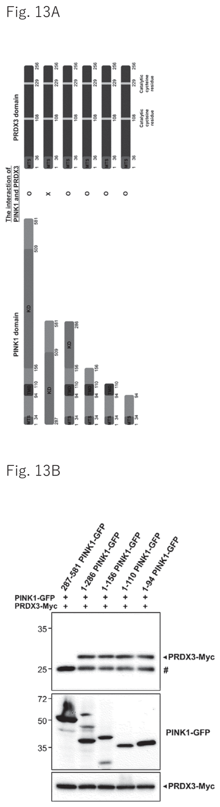

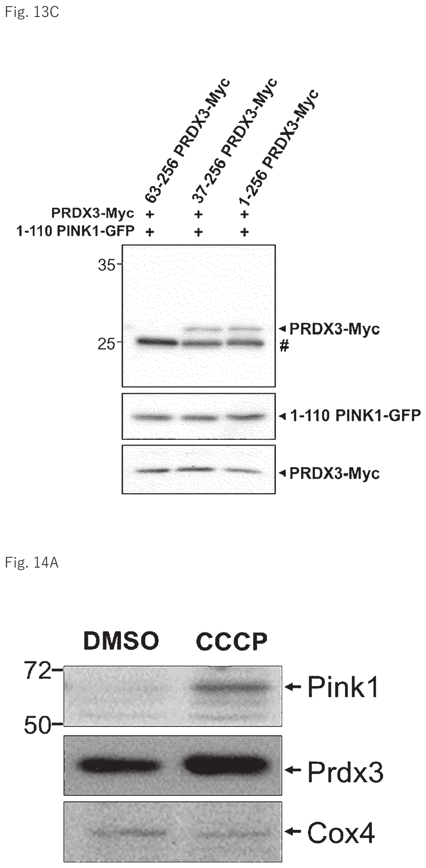

FIG. 13 shows mapping of PINK1 domain essential for binding with peroxiredoxin 3 (PRDX3). FIG. 13A shows domain boundaries of full-length PINK1 and truncated PINK1 (left) and of full-length PRDX3 (right). The PINK1 domains consist of a mitochondrial targeting sequence (MTS), a transmembrane domain (TMD), and a kinase domain. PRDX3 interacts with the N-terminus of PINK1 (1-94) containing the MTS. FIG. 13B shows immunoprecipitation with anti-GFP beads and subsequent immunoblotting with a Myc-specific antibody using WCLs from 293T cells co-transfected with various combinations of constructs expressing 287-581 PINK1-GFP, 1-286 PINK1-GFP, 1-156 PINK1-GFP, 1-110 PINK1-GFP, 1-94 PINK1-GFP, and PRDX3-Myc. Hashes indicate IgG. FIG. 13C shows immunoprecipitation with anti-GFP beads and subsequent immunoblotting with a Myc-specific antibody using WCLs from 293T cells co-transfected with various combinations of constructs expressing 63-256 PRDX3-Myc, 37-256 PRDX3-Myc, PRDX3-Myc, and 1-110 PINK1-GFP. Hash indicates IgG. Data (13B and 13C) represent three independent experiments.

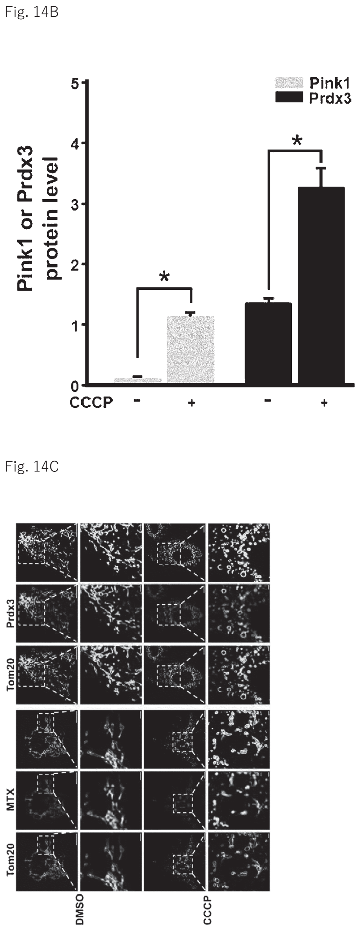

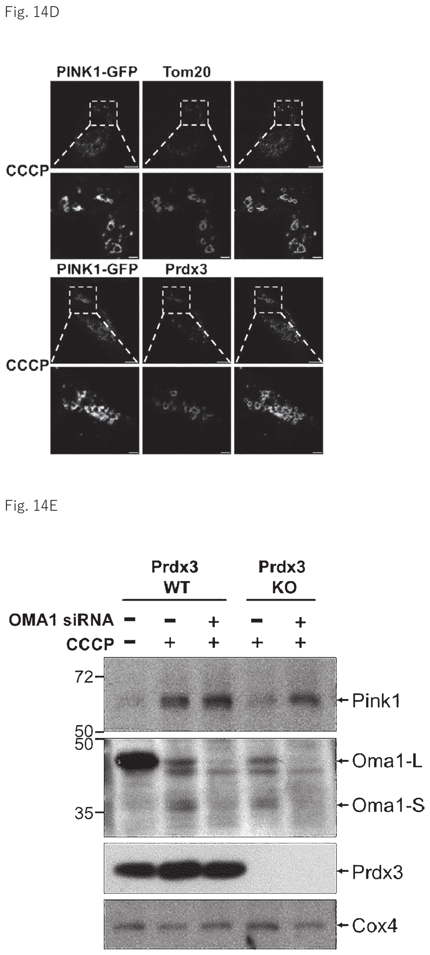

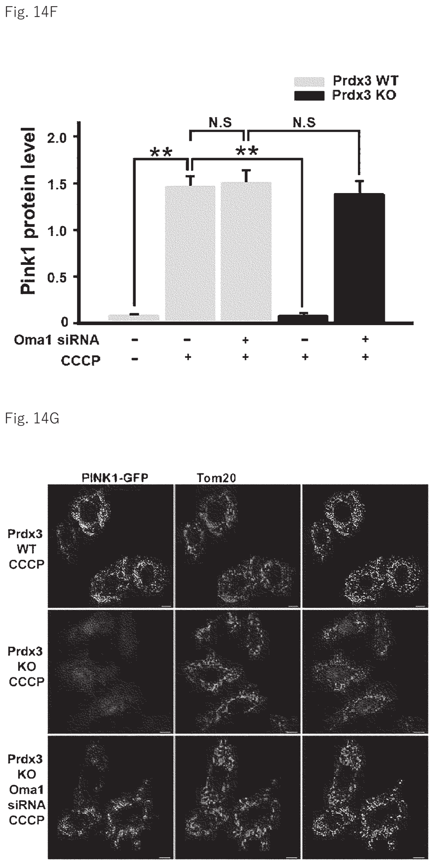

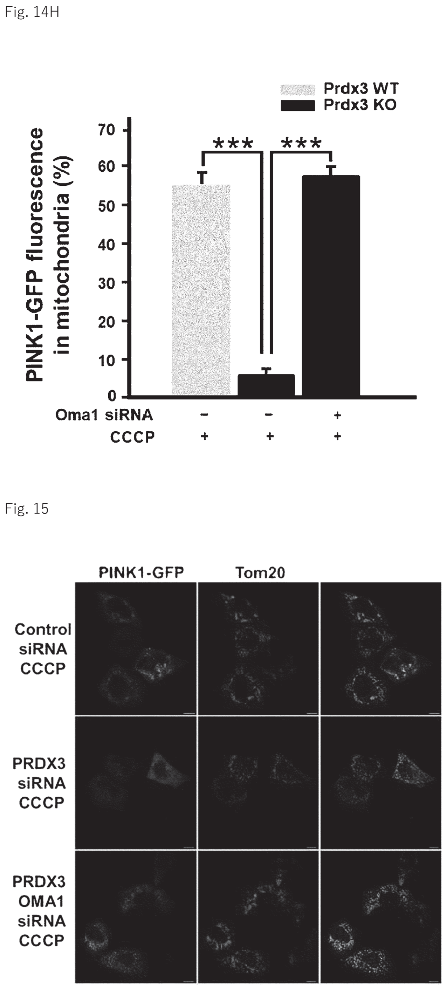

FIG. 14 shows that peroxiredoxin 3 (Prdx3) regulates the stability of PINK1. FIGS. 14A and 14B show western blot analysis (FIG. 14A) and quantification (FIG. 14B) of PINK1 and Prdx3 expression in mitochondrial fractions of MEFs treated with DMSO or 10 µM CCCP for 4 h. The mitochondrial fractions were immunoblotted with PINK1, Prdx3, and Cox4 antibodies. FIG. 14C shows immunostaining of HeLa cells transfected with pDsRed2-Mito and treated with DMSO or 10 µM CCCP for 4 h, using Tom20 and/or PRDX3 antibodies (scale bar, 2 µm (boxed areas) or 10 µm). FIG. 14D shows immunostaining of HeLa cells transfected with PINK1-GFP and treated with 10 µM CCCP for 4 h, using Tom20 and/or PRDX3 antibodies (scale bar, 2 µm (boxed areas) or 10 µm). FIGS. 14E and 14F show western blot analysis (FIG. 14E) and quantification (FIG. 14F) of PINK1 in mitochondrial fractions from Prdx3 WT and Prdx3 KO MEFs transfected with Oma1 siRNA and treated with 10 µM CCCP for 4 h. The mitochondrial fractions were immunoblotted with antibodies against PINK1, Omal, Prdx3, and Cox4. FIGS. 14G and 14H show immunostaining (FIG. 14G) using a Tom20 antibody and quantification of PINK1-GFP (FIG. 14H) in Prdx3 WT and Prdx3 KO MEFs co-infected with Oma1 siRNA and PINK1-GFP (scale bar, 10 µm; *P < 0.05 and **P < 0.01). Data (14A-14H) represent three independent experiments.
FIG. 15 shows that peroxiredoxin 3 (PRDX3) regulates the stability of PINK1, as evidenced by immunostaining of HeLa cells transfected with control, PRDX3 siRNA, or PRDX3 and OMA1 siRNAs using a Tom20 antibody (scale bar, 10 µm).

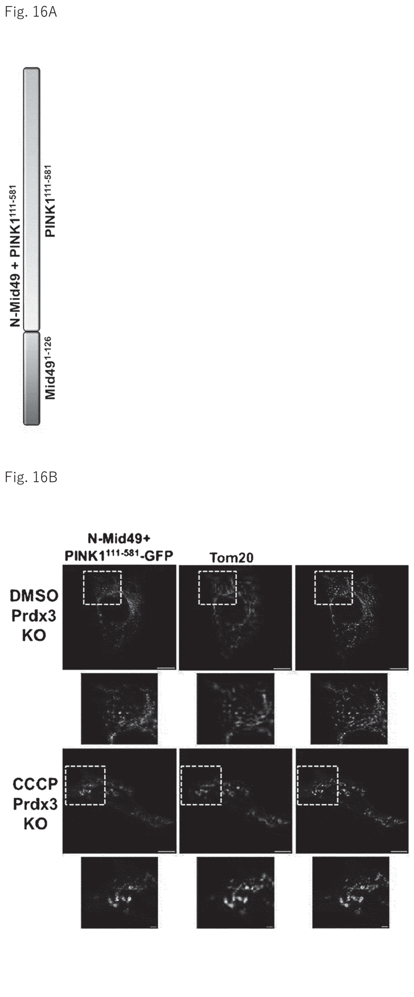

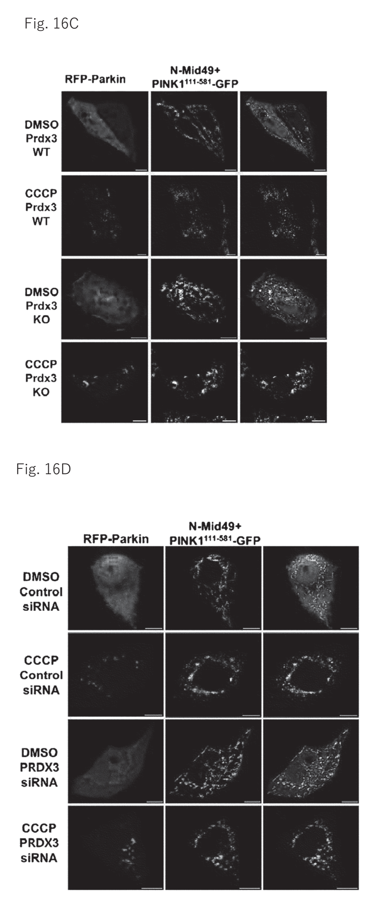

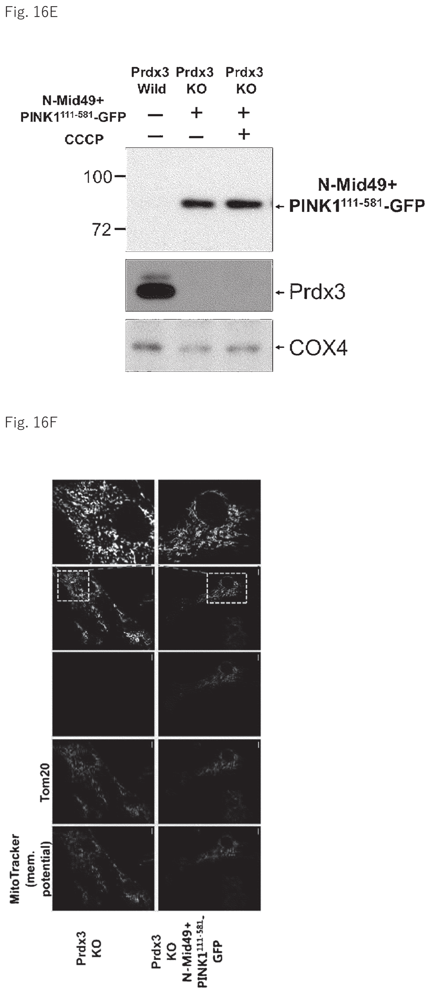

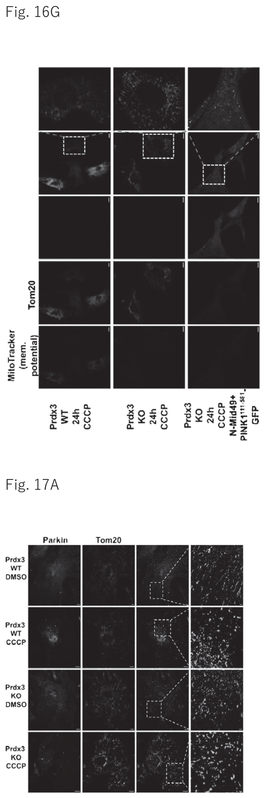

FIG. 16 shows that expression of PINK1 on the outer mitochondrial membrane (OMM) recruits Parkin to damaged mitochondria in the absence of peroxiredoxin 3 (Prdx3). FIG. 16A shows a model of the N-Mid49 + PINK1^{111_581}-GFP construct. FIG. 16B shows representative low- and high-magnification images of the boxed areas of Prdx3-deficient MEFs transfected with N-Mid49 + PINK1^{111_581}-GFP and immunostained with a Tom20 antibody (scale bar, 2 µm (boxed areas) or 10 µm). FIG. 16C shows representative images of Prdx3 WT and Prdx3 KO MEFs transfected with RFP-Parkin and N-Mid49 + PINK1^{111_581}-GFP and treated with DMSO or 10 µM CCCP for 4 h (scale bar, 10 µm). FIG. 16D shows representative images of HeLa cells transfected with control or PRDX3 siRNA, then transfected with RFP-Parkin and N-Mid49 + PINK1^{111_581}-GFP and treated with DMSO or 10 µM CCCP for 4 h (scale bar, 10 µm). FIG. 16E shows western blot analysis of mitochondrial fractions from Prdx3 WT and Prdx3 KO MEFs transfected with N-Mid49 + PINK1^{111_581}-GFP and treated with 10 µM CCCP for 4 h. The mitochondrial fractions were immunoblotted with antibodies against GFP, Prdx3, and Cox4. FIG. 16F shows MitoTracker staining or immunostaining using Tom20 antibody in Prdx3 KO MEFs transfected with N-Mid49 + PINK1111-581-GFP. FIG. 16G shows MitoTracker staining or immunostaining using Tom20 antibody in Prdx3 WT and Prdx3 KO MEFs transfected with N-Mid49 + PINK1111-581-GFP and treated with 10 µM CCCP for 24 h. (scale bar, 10 µm).

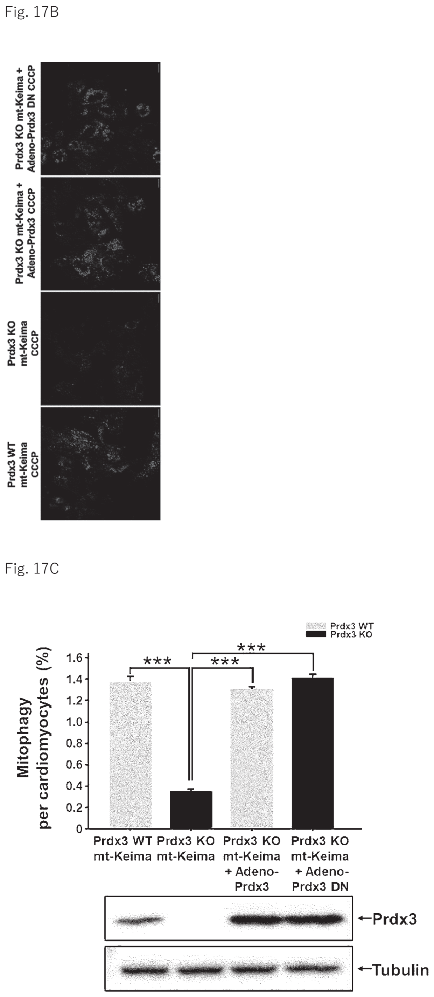

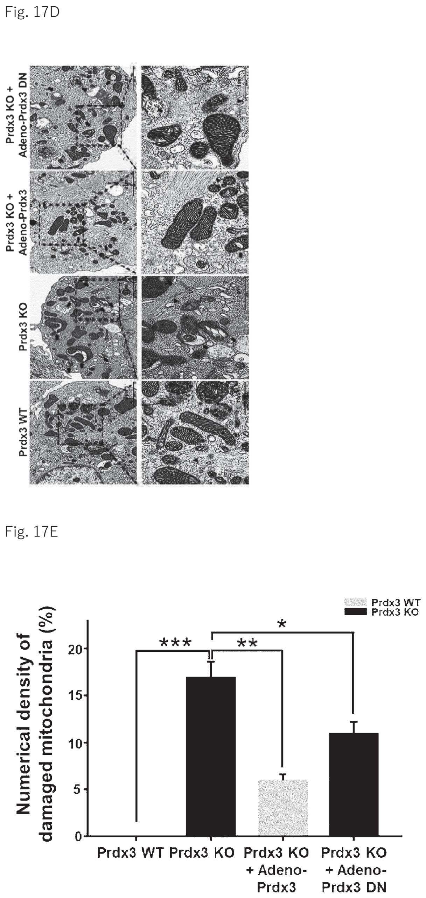

FIG. 17 shows that peroxiredoxin 3 (Prdx3) is a key regulator of mitochondrial quality control (MQC) process in cardiomyocytes. FIG. 17A shows Tom20 and Parkin immunostaining of cardiomyocytes from Prdx3 WT and Prdx3 KO mice treated with DMSO or 10 µM CCCP for 4 h (scale bar, 2 µm (boxed areas) or 10 µm). FIGS. 17B and 17C show representative images (FIG. 17B) and quantification (FIG. 17C) of mitophagy in cardiomyocytes from Prdx3 WT and Prdx3 KO mt-Keima mice. Cardiomyocytes were infected for 24 h with Prdx3 adenovirus or Prdx3 dominant-negative (DN) adenovirus and treated with 10 µM CCCP for 4 h (scale bar, 10 µm). FIGS. 17D and 17E show representative electron microscopy images (FIG. 17D) of damaged mitochondria (arrows) and quantification (FIG. 17E) in cardiomyocytes from Prdx3 WT and Prdx3 KO mt-Keima mice. Cardiomyocytes were infected for 24 h with Prdx3 or Prdx3 DN adenovirus. Arrowheads indicate myofibrils and open arrows indicate Z-lines of sarcomeres (scale bar, 1 µm (boxed areas) or 2 µm; *P < 0.05, **P < 0.01, and ***P < 0.001).

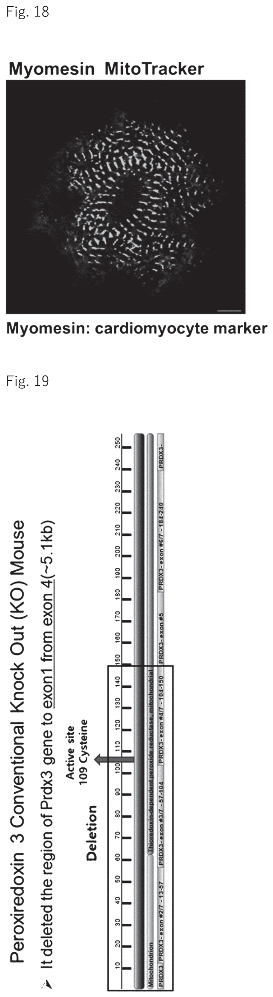

FIG. 18 shows immunostaining results of isolated neonatal cardiomyocytes using antibodies against myomesin (a cardiomyocyte marker) and MitoTracker (scale bar, 10 µm).
FIG. 19 shows the deleted gene region in a Prdx3 conventional knockout (KO) mouse.

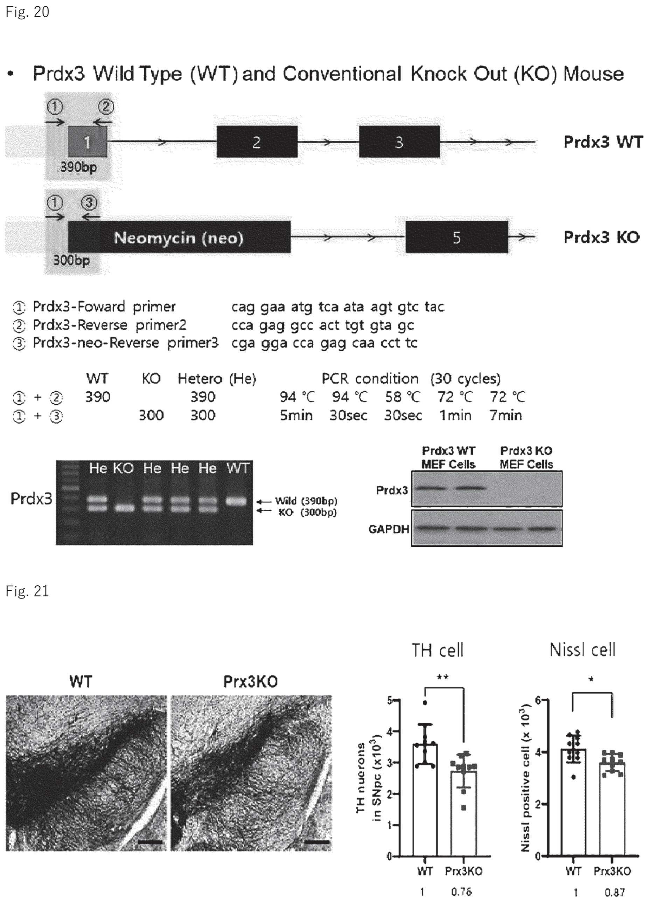

FIG. 20 shows confirmation of a Prdx3 conventional knockout mouse by PCR.
FIG. 21 shows analysis of the substantia nigra of the brain, where dopaminergic neurons are present, in 5-month-old mice.

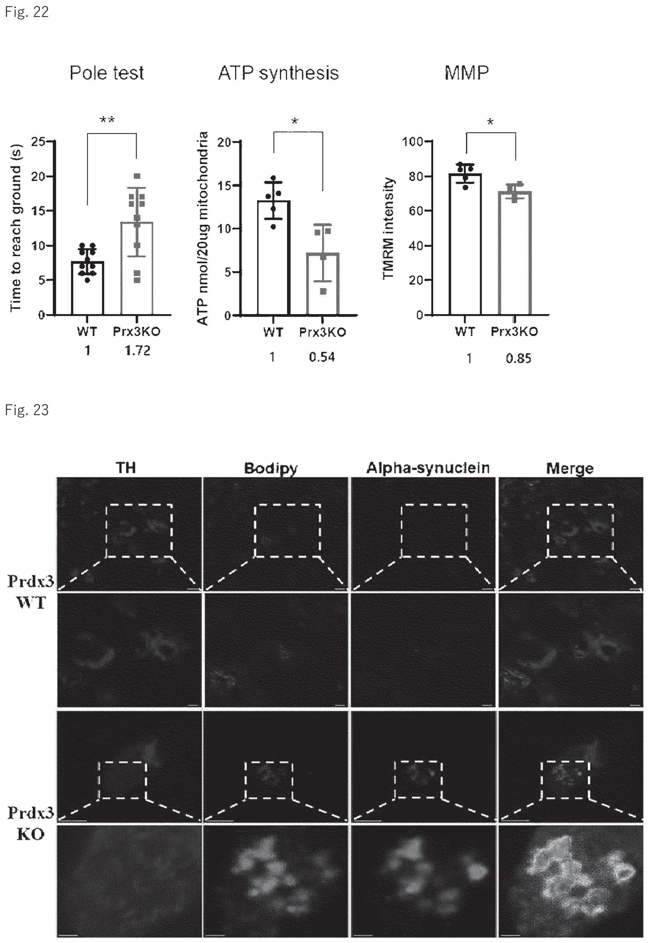

FIG. 22 shows that mitochondrial function is reduced in the substantia nigra of the brain of a Prdx3 knockout mouse.
FIG. 23 shows alpha-synuclein aggregation and lipid droplets, which are observed in Parkinson's disease, in dopaminergic neurons of the substantia nigra of a Prdx3 knockout mouse.

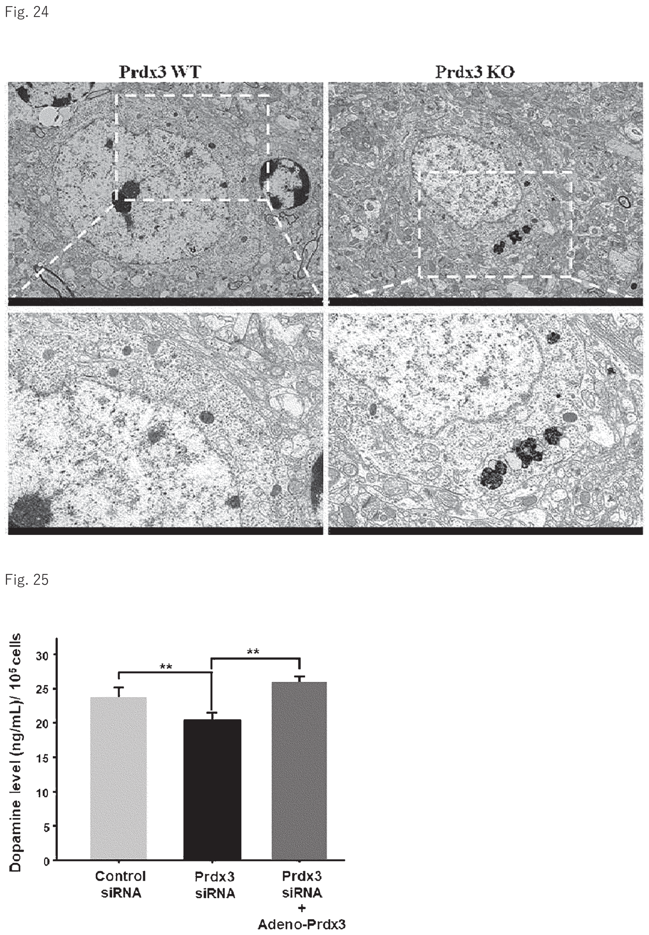

FIG. 24 shows that lipid droplets and neuromelanin, features of Parkinson's disease, are markedly increased in dopaminergic neurons of the substantia nigra of a Prdx3 knockout mouse.
FIG. 25 shows that suppression of Prdx3 expression decreases dopamine levels in SH-SY5Y neuronal culture cells, and treatment with adenovirus-Prdx3 restores dopamine levels.

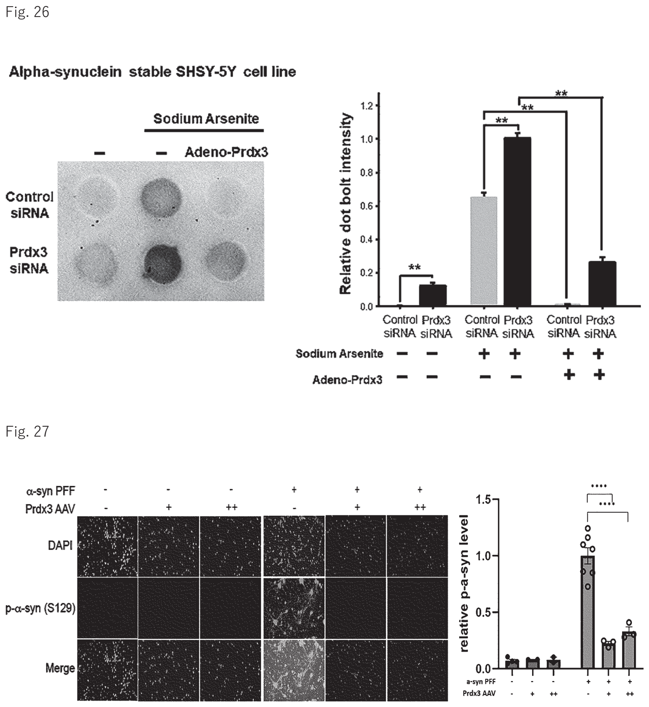

FIG. 26 shows that, under oxidative stress induced by sodium arsenite in SH-SY5Y neuronal culture cells, suppression of Prdx3 expression increases alpha-synuclein aggregation, whereas restoration of Prdx3 expression decreases alpha-synuclein aggregation.
FIG. 27 shows, in mouse neurons in which alpha-synuclein aggregation is formed by treatment with alpha-synuclein pre-formed fibrils (PFFs), that increasing Prdx3 expression by treatment with adenovirus-Prdx3 eliminates alpha-synuclein aggregation, as confirmed by immunostaining.

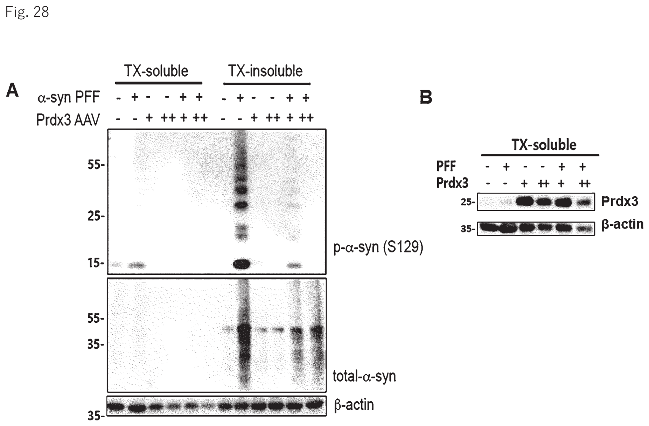

FIG. 28 shows, in mouse neurons in which alpha-synuclein aggregation is formed by treatment with alpha-synuclein pre-formed fibrils (PFFs), that western blotting demonstrates a decrease in insoluble alpha-synuclein indicative of alpha-synuclein aggregation when Prdx3 expression is increased by treatment with adenovirus-Prdx3.

### [Modes of the Invention]

Hereinafter, the present invention will be described in further detail with reference to the following examples. However, these examples are provided only to illustrate the present invention, and the scope of the invention is not limited thereto.

### Examples

### 1. Experimental materials and methods

### 1.1. Animal Models

To generate Prdx3-deficient mitochondria-targeted Keima (mt-Keima) mice, Prdx3-deficient mice were crossed with mt-Keima mice. To generate Pinkl-deficient mt-Keima mice, Pink1-deficient mice (these mice were provided by Prof. Han-Woong Lee, Yonsei University) were crossed with mt-Keima mice. Mice were backcrossed more than seven times in the C57BL/6J background (Jackson Laboratory). All animal care and experimental procedures were performed in compliance with protocols approved by the Institutional Animal Care and Use Committee of Ewha Womans University.

### 1.2. Drosophila Strains

Mt-Keima transgenic Drosophila (UAS-mt-Keima) was generated previously [17]. The white RNAi (whiteGD14981) lines were purchased from the Vienna Drosophila Resource Center. The da-GAL4 and Prx3 RNAi (Prx3HMJ22845) lines were purchased from the Bloomington Stock Center (Indiana University, Bloomington, IN, USA).

### 1.3. Mammalian Cell Cultures

Primary mouse embryonic fibroblasts (MEFs) were isolated from wild-type and Prdx3- deficient embryos on embryonic day 13.5. Primary MEFs, HeLa cells, and 293T cells were maintained in Dulbecco's modified Eagle's medium (DMEM, Invitrogen) supplemented with 10% FBS (Invitrogen), 100 U/ml penicillin, and 100 µg/ml streptomycin (Invitrogen). Cardiomyocytes were isolated from wild-type and Prdx3-deficient mice on postnatal day 3 using a Pierce^{™} Primary Cardiomyocyte Isolation Kit per the manufacturer's instructions. Isolated cells were cultured for 7 days prior to analysis to confirm the proper cell morphology. To restore the expression of Prdx3, Prdx3-deficient cardiomyocytes were infected with Prdx3 adenovirus or dominant-negative Prdx3 (Prdx-DN) adenovirus (Sirion).

### 1.4. Plasmids and RNAi oligonucleotides

Prdx3-Myc plasmids were generated via PCR amplification of full-length PRDX3 (NCBI accession number NM_006793.5) and cloning of the product into the EcoRI-XhoI sites of pcDNA.3.1/Myc-His (-) A plasmids (Invitrogen). The constructs encoding PINK1-GFP were generated via PCR amplification of full-length PINK1 (NCBI accession number NM_032409.3) and subcloning of the product into the EcoRI-BamHI sites of pEGFP-N3 plasmids (Clontech). The construct encoding Mid49-GFP was generated via PCR amplification of full-length Mid49 (isoform 1; NCBI accession number NM_139162.3) and cloning of the product into the EcoRI-BamHI sites of pEGFP-N3 plasmids (Clontech). The construct encoding the N-terminal sequence of Mid49 (amino acids 1-126, N-Mid49) or amino acids 111-581 of PINK1 (PINK1111-581) was generated via PCR amplification of full length Mid49 or PINK1, respectively. To generate the fusion construct of N-Mid49 and PINK1111-581, Mid491-126 was subcloned into the XhoI-EcoRI sites of pEGFP-N3 plasmids (Clontech), followed by subcloning of PINK1111-581 into the EcoRI-BamHI sites of N-Mid49 pEGFP-N3 plasmids. Point mutations in PRDX3 (to express PRDX3L53V and PRDX3DN) and PINK1 (to express PINK1A93V) were generated using a Phusion Site-directed Mutagenesis Kit according to the manufacturer's protocol (Thermo Fisher Scientific). The construct encoding PRKN-GFP was generated via PCR amplification of full-length PRKN (isoform 1; NCBI accession number NM_004562.3) and subcloning of the product into the EcoRI-BamHI sites of pERFP-C1 plasmids (Clontech). pDsRed2-Mito (Clontech) was used for mitochondrial matrix staining. The construct encoding GST-Δ1-40PRDX3 was created by cloning the product into the EcoRI-XhoI sites of pGEM-4T-1 plasmids (GE Healthcare). siRNAs were synthesized by GenePharma. Human siPRDX3 targeted the sequence 5'-AAG CCA AGT CCA GCT GCT TCC-3', human siOMA1 targeted the sequence 5'-GAA GTG CTT TGT CAT CTA ATT-3' and mouse siOma1 targeted the sequence 5'-GGA TAC AGT CAA AGT TGC AGG-3'. As a control, Silencer Negative Control siRNA (GenePharma) was used.

### 1.5. Transfection and Drug Treatments

To overexpress mito-catalase, Prdx3-deficient MEFs or PRDX3-depleted HeLa cells were infected with a mito-catalase adenovirus and cultured for 24 h in DMEM supplemented with 10% FBS. Cells (2 × 105 cells/well) were seeded in six-well plates for 18 h prior to transfection. Plasmid transfection was performed in OPTI-MEM medium (Invitrogen) containing 3 µl of Lipofectamine 2000 for 4 h or the NEON^{™} transfection system (Invitrogen). The cells were then seeded onto microscope dishes with cover-glass bottoms (SPL) at a density of approximately 0.5 × 105 cells/ml. For RNAi transfection, cells were seeded onto six-well plates at 30-40% confluency (or an equivalent density). Then, 4 µl of Lipofectamine 2000 (Invitrogen) or 5 µl of RNAiMAX (Invitrogen) and 40 nM RNAi oligonucleotides were added to each well. Cells were re-transfected 24 h later with 3 µl of Lipofectamine 2000 and the appropriate plasmid DNA. For mitochondrial staining, cells were treated with 100 nM MitoTracker Red CMXRos (Invitrogen), a live mitochondria-labeling fluorescent dye exhibiting membrane potential-dependent accumulation, in DMEM for 20 min prior to fixation. For live imaging of mitochondria, cells were treated with 150 nM MitoTracker Green (Invitrogen) and 5 µM MitoSOX (Invitrogen), a mitochondrial superoxide indicator in live cells, in DMEM for 20 min. For carbonyl cyanide mchlorophenylhydrazone (CCCP Sigma), oligomycin (Sigma), antimycin A (Sigma), or MG132 (Sigma) treatment, cells were seeded on poly-L-lysine-coated coverslips (0.1 mg/ml, Sigma) and incubated with 10 µM CCCP for 4-24 h, 2.5 µM oligomycin plus 250 nM antimycin A (OA) for 4 h, or 20 µM MG132 for 4 h in serum-containing medium. DMSO was used as the vehicle control.

### 1.6. Electron Microscopy

To prepare samples for cellular transmission electron microscopy (TEM), heart, liver, skeletal muscle, and brain tissues were isolated from 10-week-old wild-type and Prdx3-deficient mice. The samples were then fixed with 2% glutaraldehyde paraformaldehyde in 0.1 M PBS (pH 7.4) for 2 h and washed three times for 30 min in 0.1 M PBS (pH 7.4, 1 mM). The tissues were then post-fixed with 1% OsO4 dissolved in 0.1 M PBS (pH 7.4) for 2 h, dehydrated in a graded ethanol series (50, 60, 70, 80, 90, 95, and 100%), and incubated with propylene oxide. Specimens were embedded using a Poly/Bed 812 kit (Polysciences, USA). After being embedded in pure fresh resin at 60°C in an electron microscope oven (TD-700, DOSAKA, Japan) for 24 h, 300-nm-thick sections were initially cut and stained with toluidine blue for observation under a light microscope (Olympus BX40, Japan). Then, 80-nm-thick sections were double-stained with 7% uranyl acetate and lead citrate for contrast staining (20 min). The sections were then cut using a LEICA Ultracut UCT Ultramicrotome (Leica Microsystems, Austria). All of the samples were observed using TEM (JEM-1011, JEOL, Japan) at an acceleration voltage of 80 kV.

### 1.7. Measurement of Mitophagy Levels

Mitophagy levels were examined using the pH-dependent fluorescent probe mt-Keima via confocal microscopy as described previously. To analyze the mt-Keima fluorescence signals, mt-Keima mouse and Drosophila tissue samples were examined using a Zeiss LSM 800 confocal microscope (Carl Zeiss) equipped with Plan-Apochromat 10×/0.45 M27, Plan- Apochromat 20×/0.8 M27, and c-Apochromat 40×/1.20W Korr lenses. Mt-Keima fluorescence was imaged using two sequential excitation lasers (488 and 555 nm) and a 595-700 nm emission bandwidth. Quantitation of mitophagy based on the mt-Keima confocal images was performed using Zeiss Zen software on a pixel-by-pixel basis, as described previously. The mitophagy level (% of mitophagy) was defined as the number of pixels that had a high red/green ratio divided by the total number of pixels. To quantify the mitophagy level in heart, at least five tissue samples were used for quantification, and the average values were calculated. In all confocal microscopy analyses, all imaging parameters remained constant, and only the gain level was adjusted to avoid saturation of any pixel. The results are presented as the mean ± SD.

### 1.8. Mitochondrial Isolation

Mitochondria were isolated from MEF, 293T cell, and HeLa cell lysates using a mitochondria isolation kit for cultured cells according to the manufacturer's protocol (Thermo Fisher Scientific, Inc.).

### 1.9. Measurement of the ATP Levels and Respiration Measurements

Measurement of the ATP level in cardiomyocytes for the ATP assay, mitochondria were isolated from cardiomyocytes lysates using a mitochondria isolation kit (Thermo Fisher Scientific, Inc.). The relative ATP level was calculated by dividing the measured ATP concentration by the 20 µg mitochondria. The ATP concentration was measured by ENLITEN^{®} ATP Assay System Bioluminescence Detection Kit for ATP Measurement (Promega, USA) as previously described. Oxygen consumption rate (OCR) of cardiomyocytes was measured by XFp Analyzer (Agilent, USA) as described previously.

### 1.10. Immunoprecipitation and Western Blotting

For immunoprecipitation experiments, non-transfected and transfected cells were lysed in whole-cell extraction buffer (10 mM HEPES [pH 7.9], 400 mM NaCl, 0.1 mM EDTA, 5% glycerol, 1 mM DTT, and protease inhibitors). Non-treated cell lysate or anti-PRDX3 (1 µg) was added to the lysate followed by the addition of anti-Myc-tagged agarose (MBL) or protein A-agarose (Upstate Biotech) in TEG reaction buffer (20 mM Tris-HCl at pH 7.4, 1 mM EDTA, 10% glycerol, 1 mM DTT, and 150 mM NaCl), and the mixture was stirred for 3 h or overnight at 4°C. Immunoprecipitates were washed in TEG washing buffer (TEG reaction buffer containing 0.1% Triton X-100). For Western blotting, cells were lysed in whole-cell extract buffer or homogenized using a MICCRA D-8 homogenizer (ART Moderne Labortechnik) in protein extraction buffer (20 mM HEPES [pH 7.9], 300 mM NaCl, 10 mM EDTA, 0.1% NP40, 100 mM KCl, and protease inhibitors). Total protein was fractionated on a sodium dodecyl sulfate-polyacrylamide gel and transferred to nitrocellulose membranes (Amersham Biosciences). Primary antibodies against the following proteins were used: PRDX3 (1:300, AbFrontier, LF PA0044), catalase (1:100, Labfrontier, BC 100494), PRKN (1:100, Santa Cruz Biotechnology, sc-32282), GFP (1:100, Santa Cruz Biotechnology, sc- 9976), TOM20 (1:100, Santa Cruz Biotechnology, sc-11415), tubulin (1:300, Sigma, T6199), OMA1 (1:100, Proteintech, 17116-1-AP), PRKN (1:100, Abcam, ab77924), Myc (1:200, Abcam, ab32), COX4 (1:100, Abcam, ab33985), myomesin (1:100, DSHB, B4-S), and PINK1 (1:50, NOVUS, BC 100494). The secondary antibodies were fluorescein-conjugated anti-rabbit IgG, anti-mouse IgG, anti-goat IgG (Invitrogen) and anti-rabbit, anti-mouse, and anti-goat HRP-conjugated antibodies (Zymed Laboratories). Protein-antibody complexes were detected using the ECL Plus system (Amersham Biosciences).

### 1.11. Transthoracic Echocardiography and MI Surgery

Transthoracic echocardiography was performed using the Vevo2100 system (VisualSonics) with a 25-55-MHz linear array transducer in wild-type and Prdx3-deficient mice (10 and 49- 55 weeks old). After the induction of anesthesia with 1.5-2% isoflurane, mice were subjected to echocardiography with the heart rate maintained in the physiological range (>450 bpm) by anesthesia, and body temperature was maintained at 37°C using a homeothermic pad during echocardiography. Left ventricular (LV) variables such as the ejection fraction (EF), fractional shortening (FS), SV, cardiac output (CO), end-diastolic volume, and end-systolic volume were analyzed via the long-axis view using Simpson's monoplane method of disks approach and VevoStrain software provided by the manufacturer. Wild-type and Prdx3- deficient mice (10-12 weeks old) were subjected to cardiac surgery under anesthesia with isoflurane. The left anterior descending coronary artery was permanently occluded using 7-0 silk suture. Transthoracic echocardiography was performed 15 days after surgery using the Vevo2100 system (VisualSonics) with a 25~55-MHz linear array transducer. Anesthesia was conducted and maintained via the inhalation of 1.5-2% isoflurane, and body temperature was monitored during echocardiography. LV variables such as EF, SV, end-diastolic volume, and end-systolic volume were analyzed via the long-axis view Simpson's monoplane method of disks approach using VevoStrain software provided by the manufacturer.

### 1.12. Cardiac Fibrosis Analysis

Serial sections of Masson's trichrome-stained hearts were used to assess left ventricular (LV) fibrosis. Paraffin-embedded LV samples were sectioned into 6-µm-thick slices and stained using a Masson's Trichrome Stain Kit (BBC Biochemical) according to the manufacturer's instructions. Tissue sections were scanned using the Vectra Polaris automated quantitative pathology imaging system (PerkinElmer), and the percentage of fibrosis relative to the LV area was measured using inForm Advanced Imaging Analysis Software (PerkinElmer).

### 1.13. Live Imaging and Confocal Microscopy

The live imaging of cells was performed using a spinning disc confocal system (A1C; Nikon). For live cells, imaging was performed at 37°C and 5% CO2 in an LCI chamber (Chamlide TC; LCI), and images were taken using a ×60 numerical aperture (NA) 1.4 oil objective lens. For fixed cells, imaging was performed using a laser-scanning confocal microscope (LSM880; Carl Zeiss) and structured illumination microscope (ELYRA S.1; Carl Zeiss). Images were taken with a ×63 oil objective lens (NA 1.4). Images were analyzed using Carl Zeiss Elements, Photoshop (Adobe), IMARIS (Bitplane AG), or ImageJ (US National Institutes of Health) software. Scale bars were generated using Carl Zeiss Elements and ImageJ.

### 1.14. Analysis of Damaged Mitochondria and Mitochondrial ROS

To measure depolarized (damaged) mitochondria, only mitochondria-associated MitoTracker Red CMXRos (Invitrogen) images on consecutive z-planes were quantified using ImageJ software. To analyze mitochondrial ROS, only mitochondria-associated MitoSOX (Invitrogen) images on consecutive z-planes were quantified using ImageJ software. Statistical analysis was performed using SigmaPlot (Systat Software, Inc.). Data are presented as the mean ± SEM of at least three experiments.

### 1.15. Immunofluorescence and Histology

Cultured cells were fixed in 4% formaldehyde in PBS for 20 min at room temperature (RT), washed with PBS, and permeabilized with 0.1% Triton X-100 for 15 min. Cells were washed with PBS, blocked with 3% BSA in PBS for 1 h, and incubated with primary antibodies in PBS for approximately -1-3 h at RT. After washing with PBS, the cells were incubated with secondary antibodies for 1 h at RT. The cells were then counterstained with 10 µM 4, 6- diamidino-2-phenylindole and mounted with Vectashield (VECTOR). Paraffin-embedded sections (6 µm thick) of the heart were fixed in 10% buffered formaldehyde and used for TUNEL assays and immunofluorescence analyses.

### 1.16. Mass Spectrometry

The gel PRDX3 band was destained and digested with trypsin, and the resulting peptides were extracted and the peptides were analyzed using nanoAcquity^{™} UPLC/ESI/q-TOF MS/MS (SYNAPT^{™} G2Si HDMS^{™}, Waters Co., UK).

### 1.17. TUNEL Assay

Apoptosis in myocardial infarcted heart 24 h after MI was assessed using a TACS 2 TdT fluorescein in situ apoptosis detection kit (Trevigen) according to the manufacturer's protocols.

### 1.18. Statistical Analysis

Differences between two experimental groups were analyzed using Student's t-test or Mann- Whitney's U test. One-way ANOVA with Šidák's correction was used to compare three or more groups. P < 0.05 was considered statistically significant.

### 2. Experimental results

### 2.1. Prdx3 deficiency induces cardiac hypertrophy and dysfunction

Mitochondria contribute substantially to cardiovascular homeostasis, and their fine-tuning via MQC is crucial for cardiovascular cell survival and the maintenance of physiological cardiac function. Disruption of MQC is closely linked to cardiac defects.

To determine the role of Prdx3 in myocardial MQC, cardiac phenotypes were examined in 10- and 52-week-old wild-type and Prdx3-deficient mice. Prdx3-deficient mice exhibited cardiac hypertrophy, including significant increases of the heart weight to body weight ratio, decreased CO at 52 weeks of age, and decreased SV at both 10 and 52 weeks of age without changes of EF (FIGS. 1A to 1D and FIG. 2A). Thus, our results are consistent with previous reports that mitochondrial dysfunction is involved in heart failure caused by diastolic dysfunction, which decreases SV and CO . Therefore, the induction of LV remodeling and cardiac hypertrophy by Prdx3 deficiency suggests that mitochondrial dysfunction is a critical risk factor of heart failure, reminiscent of human HFpEF.

In addition, Prdx3-deficient mice had a similar heart size as wild-type mice at 10 weeks old but exhibited cardiac hypertrophy with significant increases cardiomyocyte size and LV fibrosis at 52 weeks old (FIGS. 1E to 1I, FIG. 2B). Damaged mitochondria in Prdx3-deficient cardiomyocytes were observed in 10-week-old mice, and their numbers were increased markedly at 52 weeks old, with the mice displaying giant and damaged mitochondria with morphologically peculiar shapes and an extremely large size (Fig. 1J and K). These results suggest that MQC dysfunction induced by Prdx3 deficiency leads to the accumulation of dysfunctional and giant mitochondria and causes cardiac dysfunction.

### 2.2. Prdx3 deficiency aggravates cardiac dysfunction associated with myocardial infarction (MI)

Mitochondrial injury caused by oxidative stress, a hallmark of infarcted cardiomyopathy, promotes excessive cardiomyocyte loss and LV remodeling .

To assess whether mitochondrial dysfunction and reduced mitophagy caused by Prdx3 deficiency exacerbates heart failure after MI, cardiac function was evaluated using echocardiography 15 days after the induction of MI (FIG. 4A). EF and SV were significantly lower in Prdx3- deficient mice than in wild-type mice. LV end-systolic volume was significantly increased in Prdx3-deficient mice, but LV end-diastolic volume was not significantly different between wild-type and Prdx3-deficient mice (FIGS. 3A to 3E). These results indicate that Prdx3 deficiency aggravates cardiac dysfunction after MI. Moreover, Masson's trichrome staining illustrated that Prdx3 deficiency exacerbated cardiac fibrosis and LV remodeling (FIG. 3F). In addition, we observed that the number of damaged mitochondria with multiple vesicles was higher in the infarcted hearts of Prdx3-deficient mice than in those of wild-type mice (FIG. 3G). Disruption of the MQC-driven elimination of damaged mitochondria leads to pernicious cell death, which is characterized by the activation of apoptotic cascades.

We performed in situ apoptosis analysis using the infarcted hearts of wild-type and Prdx3-deficient mice following MI. We observed that apoptotic cell death was significantly increased in the infarcted hearts of Prdx3-deficient mice (FIG. 3H). Although 10-week-old Prdx3-deficient mice did not exhibit obvious cardiac dysfunction under physiological conditions (FIG. 4B), an increased number of damaged mitochondria attributable to impaired MQC might explain the prevalence of heart failure after MI. Thus, under MI, Prdx3 deficiency results in increased mitochondrial damage and consequent apoptosis in infarcted hearts.

### 2.3. In vivo inhibition of mitophagy by Prdx3 deficiency

To determine further whether mitochondrial damage caused by Prdx3 deficiency results from mitochondrial ROS accumulation, we examined mitochondrial phenotypes in Prdx3-deficient mice. Mitochondrial membrane potential was analyzed in MEFs isolated from Prdx3- deficient mice, revealing increased numbers of damaged or depolarized mitochondria (FIGS. 5A and 5C). Next, an analysis of mitochondrial ROS in Prdx3-deficient MEFs revealed that Prdx3 deficiency led to increased mitochondrial ROS levels (FIGS. 5B and 5D). Given the primary molecular function of Prdx3 in preventing ROS accumulation, we next examined whether overexpression of mito-catalase, a mitochondrial ROS scavenger, could ameliorate the mitochondrial damage associated with Prdx3 deficiency. Mito-catalase overexpression appeared to reduce mitochondrial damage caused by Prdx3 deficiency (FIGS. 5A to 5E). These findings indicate that Prdx3 deficiency-induced mitochondrial damage is closely associated with aberrant ROS accumulation. Furthermore, Prdx3 deficiency in vivo causes mitochondrial damage in the heart and other tissues of Prdx3-deficient mice (FIGS. 6A and 6B). The ATP level and oxygen consumption rate(OCR) were also significantly reduced in Prdx3-deficient cardiomyocytes, demonstrating that Prdx3 deficiency causes cardiac mitochondrial dysfunction (FIGS. 7A to 7G). The numbers of damaged mitochondria were increased in the hearts, livers, skeletal muscles, and brains of 52-week-old Prdx3-deficient mice (FIGS. 1J and 1K, FIGS. 6A and 6B), suggesting that Prdx3-deficient mice represent a suitable animal model for studying MQC.

Next, to address the possible coordination between the two MQC processes in the prevention of mitochondrial damage through the regulation of ROS by mitochondria-specific Prdx3 and the elimination of damaged mitochondria via PINK1-mediated mitophagy, we compared mitochondrial phenotypes in the hearts of Prdx3- and Pink1-deficient mice. Whereas mitochondrial damage was increased in the hearts and other tissues of Prdx3-deficient mice (FIGS. 1J and 1K, FIGS. 6A and 6B), mitophagy was markedly lower in the hearts and other tissues of Prdx3-deficient mice than in wild-type and Pink1-deficient mice (FIGS. 8A and 8B, FIGS. 9A to 9C).

We next determined the pathophysiological outcome after MI, specifically whether the modality of mitophagy is influenced by Prdx3. We characterized the cardiac function of Prdx3 in mitophagy using Prdx3-deficient mt-Keima mice with MI, mitophagy was significantly increased in the hearts of wildtype mt-Keima mice 4h after MI, while it was markedly reduced in the hearts of Prdx3-deficient mt-Keima mice regardless of MI(FIG. 9D). We also found that Prdx3 deficiency significantly attenuated mitophagy in cardiac tissue 24h following MI (FIGS. 8C and 8D). Consistently, depletion of Prx3, the Drosophila ortholog of Prdx3, led to decreased mitophagy in wing disks from Drosophila larvae and the Drosophila adult fat body (FIG. 9E).

These results suggest a functional role of Prdx3 in the regulation of mitophagy in addition to its intrinsic role in preventing mitochondrial damage by regulating ROS levels.

### 2.4. Prdx3 regulates mitophagy via the Prdx3-dependent localization and degradation of PINK1 in mitochondria

To understand the contribution of Prdx3 to the regulation of mitophagy, we first examined whether PINK1 protein levels are altered by Prdx3 deficiency. To this end, we compared PINK1 protein levels between wild-type and Prdx3-deficient MEFs. PINK1 protein levels were higher in Prdx3-deficient and proteasome inhibitor MG132-treated Prdx3-deficient MEFs than in wild-type MEFs (FIGS. 10A and 10B, FIG. 11A). Because Prdx3 deficiency leads to reduced mitophagy despite the upregulation of PINK1, we next examined whether the submitochondrial localization of PINK1 is altered by Prdx3 deficiency. We overexpressed GFP-tagged PINK1 in control and Prdx3-deficient MEFs and compared its localization in mitochondria labeled with the OMM marker Tom20 using Airyscan super-resolution microscopy. In Prdx3-deficient MEFs or PRDX3-depleted HeLa cells, PINK1-GFP colocalized with Tom20 in the OMM, whereas PINK1-GFP preferentially localized to the matrix in wild-type MEFs and HeLa cells (FIG. 10C, FIG. 11B). These results suggest that Prdx3 affects the submitochondrial localization of PINK1 and thereby modulates its degradation.

We next assessed whether Prdx3 affected PINK1 expression under pro-mitophagy conditions. We treated wild-type and Prdx3-deficient MEFs with the mitochondrial uncoupling agent CCCP or the mitochondrial respiration inhibitors oligomycin and antimycin A (OA) and examined PINK1 protein levels. Previous studies indicated that CCCP or OA treatment strongly increases PINK1 accumulation in mitochondria, thereby inducing mitophagy. Interestingly, the induction of PINK1 accumulation in mitochondria by CCCP or OA treatment was almost completely abolished by Prdx3 deficiency (FIGS. 10A, 10B, 10D, and 10E).

Consistently, when we overexpressed PINK1-GFP or Parkin-GFP in MEFs, the CCCP-dependent mitochondrial accumulation of PINK1-GFP and Parkin-GFP was strongly inhibited in Prdx3-deficient MEFs even though ROS-induced mitochondrial damage linked to Prdx3 deficiency was ameliorated by mito-catalase overexpression (FIGS. 10F to 10I). In addition, when we overexpressed PINK1-GFP or Parkin-GFP in MEFs and HeLa cells, the CCCP- or OA-dependent mitochondrial accumulation of PINK1-GFP and Parkin-GFP was inhibited in Prdx3-deficient MEFs or PRDX3-depleted HeLa cells (FIGS. 11C and 11D). These results suggest that Prdx3 acts as a regulator of PINK1-Parkin-mediated mitophagy by modulating the submitochondrial localization and degradation of PINK1.

### 2.5. Prdx3 interacts with the mitochondrial targeting sequence of PINK1

We examined whether PRDX3 interacts with PINK1 using co-immunoprecipitation experiments, finding that endogenous PRDX3 binds with endogenous PINK1 (FIG. 12A).

To identify the critical binding sites between PRDX3 and PINK1, we created truncated and point-mutated constructs of PRDX3 and PINK1 and found that N-terminal domain of PINK1 is required for binding with PRDX3(FIGS. 13A and 13B). To determine further the N-terminal domain of PRDX3 critical for binding with PINK11-110, we performed IP experiments using PRDX363-256, PRDX337-256, PRDX31-256, and PINK11-110. We found that PINK11-110 binds with PRDX337-256 and PRDX31-256 but not with PRDX363-256, indicating that N-terminal sequence (amino acids 37-62) of PRDX3 is required for binding with PINK1 (FIG. 13C). Utilizing PRDX3Leu53Val and PINK1Ala93Val mutants, we observed that Leu53 in PRDX3 and Ala93 in PINK1 are critical for the binding between PRDX3 and PINK1 (FIGS. 12B and 12C). Dominant-negative PRDX3Cys108Ser/Cys229Ser, which blocks ROS removal by PRDX3, could still bind to PINK1, indicating that ROS-counteracting activity remained functionally intact in dominant-negative PRDX3 (FIG. 12B). GST pull-down experiments confirmed that Leu53 in PRDX3 and Ala93 in PINK1 are critical for the direct binding between these proteins (FIGS. 12D and 12E).

It has been reported that the N-terminal sequence (amino acids 1-62) of PRDX3 serves as a mitochondrial targeting signal. Mass spectrometric analysis to further clarify the mitochondrial targeting signal sequences of PRDX3 predicted that the N-terminal sequence (amino acids 1-36) of PRDX3 is essential for mitochondrial targeting and revealed that mature PRDX3 contains Leu53, ensuring its binding to PINK1 (FIGS. 12F and 12G). Crystallography of mitochondrial Prdx, the Leishmania infantum ortholog of Prdx3, illustrated that the N-terminal sequence outside the mitochondrial targeting sequence is critical for the formation of structurally stable mitochondrial Prdx. Thus, we demonstrated that the N-terminal sequence (amino acids 37-62) of PRDX3 binds to the mitochondrial targeting sequence of PINK1.

### 2.6. Prdx3 regulates PINK1 stability via its inhibition of Omal during mitophagy

We examined whether Prdx3 protein expression changes as PINK1 levels increase in damaged mitochondria. We found that Prdx3 protein levels also increased in damaged mitochondria, mimicking the upregulation of PINK1 (FIGS. 14A and 14B). We also investigated whether the submitochondrial localization of Prdx3 switches from the mitochondrial matrix to the OMM upon binding with PINK1 under CCCP-induced mitochondrial damage. Co-immunostaining of Prdx3 with Tom20 revealed that Prdx3 localized to the mitochondrial matrix in healthy mitochondria, whereas it translocated to the OMM in damaged mitochondria, as confirmed by its co-localization with either Tom20 or PINK1 (FIGS. 14C and 14D).

Diverse cellular stresses can drive mitochondrial dysfunction, which can itself promote the activation of the IMM-resident stress-induced protease OMA1. Upon activation in damaged mitochondria, Omal cleaves Dele1 and initiates a stress-induced proteolytic cascade. A recent study reported that OMA1 degrades Parkinson's disease related PINK1 mutants that are imported into damaged mitochondria. Prdxs, including Prdx3, serve dual functions as peroxidases under normal conditions and as chaperones upon exposure to diverse stresses. Human PRDX3 acts as a self-assembling chaperone that is catalytically active under stress conditions.

To determine whether PINK1 is degraded by Omal regardless of the presence of Prdx3, we examined the stability of PINK1 in damaged mitochondria in wild-type and Prdx3-deficient MEFs following Omal depletion and CCCP treatment. In damaged mitochondria undergoing mitophagy, PINK1 was not degraded by Omal in the presence of Prdx3 (FIGS. 14E and 14F). Interestingly, PINK1 was degraded by Oma1 in the absence of Prdx3, but its levels were restored by Oma1 depletion using siRNA (siOma1). PINK1-GFP was also completely lost in Prdx3-deficient MEFs and PRDX3- depleted HeLa cells after CCCP treatment. However, Omal depletion using siOma1 dramatically restored PINK1-GFP levels in cells treated with CCCP (FIGS. 14G and 14H, FIG. 15). Thus, the binding of Prdx3 to the N-terminus of PINK1 protects the latter protein against degradation by blocking its cleavage by Omal. This indicates that Prdx3 acts as a chaperone for PINK1.

To confirm that Prdx3 regulates PINK1-Parkin-mediated mitophagy by modulating the stability of PINK1 in damaged mitochondria, we performed PINK1 recruitment/rescue experiments. Mid49 regulates the mitochondrial recruitment of Drp1, which controls fission in mitochondrial dynamics, and it is an OMM protein with an N-terminal mitochondrial targeting sequence and OMM domain. Using N-Mid49, which contains the mitochondrial targeting sequence and OMM domain, we created a mitochondrial targeting domain truncated form of PINK1 (PINK1111-581) that lacked both the PRDX3-binding region and OMA1 cleavage site and localized persistently to the OMM (FIG. 16A). When N-Mid49-PINK1111-581 was overexpressed in Prdx3-deficient MEFs, the construct localized normally to mitochondria irrespective of CCCP-induced mitochondrial damage (FIG. 16B). When N-Mid49-PINK1111-581 was overexpressed in Prdx3- deficient MEFs or PRDX3-depleted HeLa cells, the construct only recruited Parkin to damaged mitochondria following CCCP treatment (FIGS. 16C and 16D). N-Mid49-PINK1111-581 was expressed stably in damaged mitochondria regardless of the presence of Prdx3 and CCCP-induced mitochondrial damage (FIG. 16E). Moreover, overexpression of N-Mid49-PINK1111-581 in Prdx3-deficient MEFs resulted in decreased mitochondrial depolarization via the occurrence of normal mitophagy compared to the findings in Prdx3-deficient MEFs (FIG. 16F). To induce mitochondrial depolarization in most cells, Prdx3-deficient MEFs and N-Mid49-PINK1111-581-overexpressing Prdx3-deficient MEFs were treated with CCCP for 24 h. The number of depolarized mitochondria was increased in Prdx3-deficient MEFs, indicating defective mitophagy. However, mitochondrial depolarization following CCCP treatment was reduced in N-Mid49-PINK1111-581-overexpressing Prdx3-deficient MEFs, demonstrating the clearance of damaged mitochondria via N-Mid49-PINK1111-581-mediated mitophagy (FIG. 16G). Thus, Prdx3 maintains PINK1 stability in damaged mitochondria by acting as a chaperone and binding with the protein, thus protecting it from cleavage by activated Omal.

### 2.7. Prdx3 regulates MQC processes in cardiomyocytes

To determine whether the suppression of mitophagy in the Prdx3-deficient heart has a functional link with PINK1-Parkin-mediated mitophagy, we examined the localization of Parkin in damaged mitochondria in wild-type and Prdx3-deficient cardiomyocytes after CCCP treatment. We found that endogenous Parkin localized to damaged mitochondria in wild-type cardiomyocytes, but not in Prdx3-deficient cardiomyocytes (FIGS. 17A and 18), indicating that Prdx3 regulates PINK1-Parkin-mediated mitophagy in cardiomyocytes.

Next, we examined whether either Prdx3 or Prdx3-DN adenovirus can rescue mitophagy under CCCP-induced mitophagy. Prdx3-deficient cardiomyocytes were infected with adenovirus carrying Prdx3 or Prdx3-DN following CCCP treatment. Prdx3-DN is defective for ROS removal but functional for PINK1-mediated mitophagy. Prdx3 or Prdx3-DN expression restored mitophagy in Prdx3-deficient cardiomyocytes to a level similar to that in wild-type cardiomyocytes (FIGS. 17B and 17C). Additionally, the number of damaged mitochondria in Prdx3-deficient cardiomyocytes was decreased by adenoviral Prdx3 or Prdx3-DN transfection, suggesting that increased mitochondrial damage upon Prdx3 deficiency can be reduced by functional mitophagy activated by Prdx3-DN with defective ROS removal (FIGS. 17D and 17E).

Taken together, Prdx3 acts as a key regulator via the interplay between the prevention of mitochondrial damage and the elimination of damaged mitochondria, and it is essential for cardiac function.

### 2.8. Effects of Prdx3 in Parkinson's Disease

### 2.8.1. Generation of Prdx3 Knockout Mice

The Prdx3 gene is located on chromosome 19 in mice and consists of exons 1 through 7, which through gene translation form a protein composed of 257 amino acids. We confirmed that the mitochondrial targeting signal exists in exon 1 of the Prdx3 gene, and that cysteine residues responsible for removing ROS are located at position 109 in exon 4 and position 230 in exon 6. The Prdx3 gene is localized in the mitochondrial matrix and functions to remove ROS from mitochondria.

A Prdx3 conventional knockout mouse was generated by introducing a construct in which a portion of the intron of the 5'-UTR of exon 1 of the Prdx3 gene and a portion of the intron of the 3'-UTR of exon 4 were recombined with the 5'-UTR of the exogenous gene neomycin into mouse embryonic stem cells using an electroporation method, thereby inducing deletion of exons 1 to 4 by homologous recombination. After selecting embryonic stem cells with deletion of exons 1 to 4 in medium containing neomycin, these cells were microinjected into mouse blastocyst embryos to generate Prdx3 knockout mice (FIG. 19). Prdx3 wild-type (WT) mice were confirmed by PCR, as shown in FIG. 20, using (i) a Prdx3 forward primer (forward primer: cag gaa atg tca ata agt gtc tac, SEQ ID NO: 4) designed from the intron region upstream of exon 1, and (ii) a Prdx3 reverse primer (reverse primer: cca rag gcc act tgt gta gc, SEQ ID NO: 5) designed from exon 1, producing a 390 base pair product. For identification of Prdx3 knockout mice, as shown in FIG. 20, PCR was performed using (i) the Prdx3 forward primer (SEQ ID NO: 4) designed from the intron upstream of exon 1 and (iii) the Prdx3 neo-reverse primer (neo-reverse primer: cga gga cca rag caa cct tc, SEQ ID NO: 6) designed from the exogenous neomycin gene introduced to delete exons 1-4 (SEQ ID NOs: 7-10), producing a 300 base pair product.

By this method, Prdx3 knockout mice were identified, and MEF (mouse embryonic fibroblast) cells derived from Prdx3 knockout mice were confirmed by western blot analysis to lack Prdx3 expression compared with Prdx3 wild-type mice (FIG. 20).

### 2.8.2. Verification of Parkinson's Disease Onset in Prdx3 Knockout Mice

Analysis of the substantia nigra of the brain containing dopaminergic neurons in 5-month-old mice revealed a significant decrease in dopaminergic neurons in the substantia nigra of Prdx3 knockout mice compared to wild-type mice (FIG. 21). Prdx3 knockout mice exhibited reduced motor activity and impaired mitochondrial function in the substantia nigra (FIG. 22). In addition, alpha-synuclein aggregation and lipid droplets, which are features of Parkinson's disease, were observed in dopaminergic neurons of the substantia nigra in Prdx3 knockout mice (FIG. 23). Electron microscopy further showed a marked increase in lipid droplets and neuromelanin in dopaminergic neurons of the substantia nigra in Prdx3 knockout mice, which are characteristic features of Parkinson's disease (FIG. 24).

These results indicate that Prdx3 knockout mice are suitable Parkinson's disease model mice for studying disease onset and therapy.

### 2.8.3. Therapeutic Effects of Prdx3 in Parkinson's Disease

In SH-SY5Y neuronal cell cultures commonly used for Parkinson's disease research, inhibition of Prdx3 expression reduced dopamine levels, while treatment with adenovirus-Prdx3 restored dopamine levels (FIG. 25).

In alpha-synuclein stable SH-SY5Y neuronal cell cultures treated with sodium arsenite to induce oxidative stress, alpha-synuclein aggregation, a direct factor in Parkinson's disease onset, was observed. Inhibition of Prdx3 expression increased alpha-synuclein aggregation, while restoration of Prdx3 expression by adenovirus-Prdx3 treatment decreased alpha-synuclein aggregation (FIG. 26).

Furthermore, treatment of neurons with alpha-synuclein pre-formed fibrils (PFFs), directly involved in Parkinson's disease pathogenesis, induced alpha-synuclein aggregation and neuronal degeneration, thereby forming an in vitro Parkinson's disease model. Immunocytochemistry confirmed that adenovirus-Prdx3 treatment, which increased Prdx3 expression, eliminated alpha-synuclein aggregation in these neurons (FIG. 27).

In addition, western blot analysis confirmed that adenovirus-Prdx3 treatment reduced insoluble alpha-synuclein, indicative of alpha-synuclein aggregation (FIG. 28). These results confirm that downregulation of Prdx3 contributes to the formation of alpha-synuclein aggregation, a critical cause of Parkinson's disease onset, and that Prdx3 itself can play an important role in Parkinson's disease therapy.

Collectively, these findings suggest that Prdx3 can be usefully employed as a therapeutic agent for Parkinson's disease.

## Claims

1. A pharmaceutical composition for preventing or treating a mitochondria-related disease, comprising a Prdx3 peptide or an analogue thereof as an active ingredient.

2. The pharmaceutical composition of claim 1, wherein the Prdx3 analogue is a dominant-negative (DN) mutant of Prdx3.

3. The pharmaceutical composition of claim 1, wherein the Prdx3 peptide removes mitophagy.

4. The pharmaceutical composition of claim 3, wherein the Prdx3 peptide regulates mitophagy through degradation of a PINK1 peptide.

5. The pharmaceutical composition of claim 1, wherein the Prdx3 peptide removes an alpha-synuclein aggregate.

6. The pharmaceutical composition of claim 1, wherein the mitochondria-related disease is selected from the group consisting of cardiovascular disease, lymphoma, glomerulonephritis, osteoporosis, motor neuron disease, muscular atrophy, Down syndrome, carcinoma, Pick disease, Parkinson syndrome, and Alzheimer's disease.

7. A health functional food for preventing or improving a mitochondria-related disease, composition comprising a Prdx3 peptide or an analogue thereof as an active ingredient.

8. A pharmaceutical preparation for preventing or treating a mitochondria-related disease, comprising a Prdx3 peptide or an analogue thereof as an active ingredient.

9. A method for preventing or treating a mitochondria-related disease, comprising administering a pharmaceutical composition comprising a Prdx3 peptide or an analogue thereof to a subject.

10. A Prdx3 knockout mouse model in which exon 1 to exon 4 in a 5'-UTR region in a Prdx3 gene are deleted.

11. The mouse model of claim 10, wherein the mouse model is a Parkinson's disease model.

12. A method for preparing a Prdx3 knockout mouse model, comprising deleting exon 1 to exon 4 in a 5'-UTR region in a Prdx3 gene.

13. The method of claim 12, wherein the deletion of exon 1 to exon 4 of the 5'-UTR region in the Prdx3 gene is performed by homologous recombination.

14. The method of claim 12, wherein the deletion is carried out by replacing a nucleotide sequence comprising exon 1 to exon 4 of the 5'-UTR region in the Prdx3 gene with a construct comprising a neomycin gene.

15. The method of claim 14, wherein the replacement with the construct is carried out by electroporation.
